# EUROPEAN PATENT APPLICATION

(11) **EP 4 785 935 A1**
(43) Date of publication of application: **05.08.2026**
(21) Application number: 24870562.6
(22) Date of filing: 19.09.2024
(51) Int. Cl.: A61K 9/50, C12N 5/10, A61P 27/02

(54) **PREPARATION METHOD AND APPLICATION OF TARGETED INTRACELLULAR NANOVESICLE**

(30) Priority: 26.09.2023 CN 202311252113
(71) Applicant: Goodwill Medical Engineering Technology (Tianjin) Co., Ltd., Tianjin 300192 (CN)
(72) Inventor: ZHANG, Xiaomin, Tianjin 300392 (CN); ZHANG, Hui, Tianjin 300392 (CN); ZHANG, Xu, Tianjin 300392 (CN); YUE, Jianhui, Tianjin 300392 (CN); YU, Gengnan, Tianjin 300392 (CN)
(74) Representative: Gassner, Birgitta
(86) International application number: PCT/CN2024/119744
(87) International publication number: WO 2025/067018

(57) **Abstract**

A preparation method and application of a targeted intracellular nanovesicle. The intracellular nanovesicle has a large yield, a small particle size and a narrow particle size distribution range, has higher encapsulation efficiency and drug loading efficiency when used as a carrier to load a drug, and has a wider distribution range and degree in a mode of intravitreal injection, for example, the drug loaded in the mode of intravitreal injection can be absorbed more quickly, and the intracellular nanovesicle has extremely good application and research value in the field of medicine. Particularly, targeting performance is given to the intracellular nanovesicle, and a targeting peptide is carried in the intracellular nanovesicle by modifying Lamp2b to obtain the intracellular nanovesicle having the targeting performance. The nanovesicle has a large yield and high targeting performance, can exert a therapeutic effect or a drug carrier effect more efficiently, and has an extremely good application prospect and research value.

## Description

### TECHNICAL FIELD

The present disclosure relates to the field of biotechnology, specifically to a method for preparing intracellular-derived targeted nanovesicles and uses thereof, especially use thereof in ocular diseases.

### BACKGROUND

Cellular secretions released from cells into the extracellular space or plasma and entering the blood circulation, known as "extracellular vesicles" (EVs), have received extensive attention and research. "Extracellular vesicles" are primarily classified into three categories: exosomes, microvesicles or microparticles, and apoptotic bodies, with exosomes being the primary component. Currently, exosomes are widely used in the treatment of various injury repairs and immune diseases. However, the cell targeting ability of exosomes still needs further improvement to reduce side effects and enhance therapeutic efficacy. Moreover, the efficiency of cellular secretion of exosomes is limited, and collecting a certain amount of exosomes requires significant time investment. Therefore, further improving their targeting ability, biosafety, and yield are issues that need to be addressed in the future.

Modification strategies for cellular nanovesicles include genetic engineering and chemical modification. Using genetic engineering to fuse the gene sequence of a target protein or polypeptide with the gene sequence of a selected exosome surface protein enables the expression of the target protein or guiding peptide on the exosome surface. Lysosome-associated membrane protein 2b (Lamp2b) is a member of the Lamp family and is a membrane protein that may be used to carry targeting proteins or polypeptides.

Furthermore, a large number of nanovesicles exist within cells, moving among various membrane-rich organelles, mediating intracellular material transport and cellular secretion pathways. Among these, constitutive secretory vesicles are the primary type of intracellular vesicles (IVs), which are produced by the endoplasmic reticulum, and exchange materials with the Golgi apparatus, lysosomes, and cell membrane, mediating the production, transport, and secretion of various secretory proteins within the cells. Their contents are abundant, containing a large number of secretory proteins, lipids, and nucleic acid molecules. To date, there have been no reports on how to enrich and apply the intracellular nanovesicles. Furthermore, our preliminary findings indicate that compared to EVs, these intracellular nanovesicles have a smaller particle size, higher content of the transmembrane protein Lamp2b, and exhibit higher drug delivery efficiency, making them more suitable for disease treatment and drug delivery.

Autoimmune uveitis is a common blinding eye disease, mainly affecting the uvea and retina. It can cause various ocular complications such as cataracts, glaucoma, maculopathy, and optic atrophy, leading to severe vision impairment. CD4-positive T helper cells (Th cells), especially IFN-γ-secreting Th1 cells and IL-17A-secreting Th17 cells, are the primary effector T cells of autoimmune uveitis, and their abnormal activation plays a crucial pathogenic role in disease occurrence. CD40L belongs to the TNF superfamily and is considered an early activation marker of CD4+ T lymphocytes. CD40/CD40L is involved in triggering multiple downstream immune signaling pathways. Early studies have demonstrated that CD40/CD40L costimulatory signaling is necessary for generating T cell-dependent antibody responses and memory B cell differentiation. Upon activation of CD40/CD40L signaling in macrophages and dendritic cells (DCs), cytokine secretion and surface activation molecule expression are induced, participating in the regulation of cross-stimulation and activation of CD4+ T cells and CD8+ T cells. Dysregulated expression of CD40/CD40L signaling pathway is also observed in various autoimmune diseases, which may promote the initiation or maintenance of pathogenic autoimmune responses. Therefore, targeting the CD40/CD40L pathway holds promise as a novel therapeutic strategy for autoimmune diseases.

Pathological retinal neovascularization (RNV) is a key pathological change in several common blinding retinal diseases, including diabetic retinopathy (DR), diabetic macular edema, retinal vascular occlusion, and retinopathy of prematurity (ROP), etc. Current clinical treatments include intravitreal injection of anti-VEGF drugs, retinal photocoagulation, and vitrectomy. Although intravitreal injection of anti-VEGF drugs can inhibit the further formation of retinal and choroidal NV, its therapeutic efficacy remains controversial. Approximately 30% of patients respond poorly to anti-VEGF drug therapy, and the effectiveness of laser and surgical treatments is limited, with a considerable number of patients losing visual function. Therefore, there is a need to explore more effective and safer alternative approaches.

Previous studies have shown that mesenchymal stem cells (MSCs) have effects such as promoting wound repair and providing neuroprotection, and are widely used in therapeutic research for retinal and neurological diseases. However, their clinical translation and application are limited by the disadvantage of cell proliferation. MSC-derived vesicles possess the biological characteristics of stem cells, are anucleate and free of genetic material, and hold significant potential for translational applications. Modifying vesicles to confer targeting capability can improve their bioavailability. Therefore, it is of great significance to specifically modify intracellular nanovesicles to endow them with targeting capability for research on autoimmune uveitis and retinal neovascular diseases, and identifying drugs for treating autoimmune uveitis and retinal neovascular diseases.

### SUMMARY

To overcome the shortcomings of the prior art, the present disclosure provides a method for preparing intracellular-derived targeted nanovesicles and uses thereof, particularly their use as drug carriers with targeting functionality.

In a first aspect of the present disclosure, an intracellular-derived targeted nanovesicle is provided, which overexpresses a targeting molecule-Lamp2b fusion protein.

Specifically, the targeting molecule includes but is not limited to a polypeptide, an antibody, an activating or inhibitory receptor, a protein ligand, a biologically active enzyme, a nucleic acid drug, or a combination thereof; in particular, the targeting molecule is the polypeptide.

In some embodiments of the present disclosure, the targeting molecule is an A25 polypeptide, and the intracellular nanovesicle can target T cells.

In some embodiments of the present disclosure, the targeting molecule is an RGD polypeptide, and the intracellular nanovesicle can target neovascularization (e.g., retinal neovascularization).

Specifically, the targeting molecule is located at N-terminus of the Lamp2b.

Specifically, a structure of the fusion protein comprises:
1) N-terminus of Lamp2b - targeting molecule - C-terminus of Lamp2b; and
2) targeting molecule - N-terminus of Lamp2b.

Specifically, the vesicle is spherical or cup-shaped.

Specifically, the vesicle has an average particle size of 50 nm -100 nm (e.g., 50, 55, 60, 65, 70, 75, 80, 85, 90, 95, 100 nm), particularly 65 nm -85 nm.

Specifically, the cell is derived from a mammal, particularly a human.

Specifically, the cell is a eukaryotic cell, particularly at least one selected from the group consisting of a stem cell, a cardiomyocyte, a human embryonic kidney cell (293T), a macrophage, a T cell, and a tumor cell; more specifically, the stem cell is a mesenchymal stem cell (MSC), including but not limited to: an umbilical cord mesenchymal stem cell (UC-MSC), a bone marrow mesenchymal stem cell (BM-MSC), an adipose-derived mesenchymal stem cell (AD-MSC), a dental pulp mesenchymal stem cell, and mesenchymal stem cells derived from placenta, amniotic fluid, and amnion.

In some embodiments of the present disclosure, the cell is a mesenchymal stem cell, particularly an umbilical cord mesenchymal stem cell (UC-MSC).

In some embodiments of the present disclosure, the cell is an epithelial cell, e.g., a 293T cell.

In some embodiments of the present disclosure, the cell is a tumor cell, e.g., a HeLa cell.

Specifically, the vesicle is prepared by the method according to the second aspect of the present disclosure.

In a second aspect of the present disclosure, a method for preparing the aforementioned vesicle is provided.

Specifically, the method comprises the steps of subjecting a cell overexpressing a targeting molecule-Lamp2b fusion protein to ultrasonic treatment, centrifugation, and ultracentrifugation sequentially.

Specifically, the method comprises the following steps:
(1) introducing a nucleotide encoding the targeting molecule-Lamp2b fusion protein into a cell;
(2) dispersing a resulting cell obtained from step (1) in a suspending solvent and performing ultrasonic treatment;
(3) subjecting a resulting liquid obtained from step (2) to one or more rounds of centrifugation, discarding cell membrane and organelle fragments, and collecting a resulting supernatant; and
(4) subjecting the supernatant obtained from step (3) to ultracentrifugation, and collecting a resulting pellet as the intracellular nanovesicle;
optionally, (5) resuspending the pellet obtained from step (4).

Specifically, methods for introducing the nucleotide encoding the targeting molecule-Lamp2b fusion protein into the cell in step (1) may comprises a method such as transformation, transduction, transfection, or infection. The nucleotide may be introduced into the cells via a vector, wherein the vector comprises a plasmid vector and a viral vector. The viral vector may be, for example, a retroviral vector, a lentiviral vector, or other vectors (e.g., an adenoviral vector, an adeno-associated viral vector).

In some embodiments of the present disclosure, step (1) comprises: constructing a plasmid overexpressing the targeting molecule-Lamp2b fusion protein, then packaging the plasmid into a lentivirus, and transfecting the cell with the lentivirus.

In one embodiment of the present disclosure, the step of constructing the overexpression plasmid comprises: inserting a cDNA fragment encoding A25 polypeptide and Lamp2b downstream of a CMV promoter in a vector plasmid.

Specifically, the cell in step (2) is an isolated cell obtained after culturing, digestion, and washing (which, through steps such as discarding cell culture medium, digestion, and washing, can exclude the possibility of isolating and obtaining an extracellular vesicle).

Specifically, the method may further comprise steps of cell digestion and counting; in some embodiments of the present disclosure, the step of cell digestion comprises: culturing cells until they reach 90% confluence, discarding cell culture medium, washing the cells, adding trypsin to digest the cells, and then neutralizing and washing the cells.

Specifically, a cell density in the suspending solvent is 1-4×10⁶ cells/mL, for example, 1×10⁶ cells/mL, 2×10⁶ cells/mL, 3×10⁶ cells/mL, or 4×10⁶ cells/mL. In some embodiments of the present disclosure, the cell density is 1×10⁶ cells/mL.

Specifically, the suspending solvent is any buffer suitable for cell culture, such as PBS, a Tris buffer, or a glycine buffer. In some embodiments of the present disclosure, the suspending solvent is PBS.

Specifically, an amplitude of the ultrasonic treatment in step (2) is 20%-35% (e.g., 20%, 25%, 30%, 35%), for example, 20%-30%.

Specifically, the amplitude of the ultrasonic treatment in step (2) is 20%-25% (e.g., 20%, 22%, 24%, 25%); in some embodiments of the present disclosure, the amplitude of the ultrasonic treatment is 20%.

Specifically, the ultrasonic treatment in step (2) is performed for 10 s -60 s (e.g., 10, 15, 20, 25, 30, 60 s), for example, 10 s -30 s.

Specifically, the ultrasonic treatment in step (2) is performed for 10 s -20 s (e.g., 10, 15, 18, 20 s), preferably 15 s; in some embodiments of the present disclosure, the ultrasonic treatment is performed for 15 s, with a cycle of 2 s on and 2 s off.

In some embodiments of the present disclosure, the centrifugation in step (3) is performed twice, with respective parameters as follows:
1000 g -3000 g (e.g., 1000, 1500, 2000, 2500, 3000 g) for 5 minutes -20 minutes (e.g., 5, 8, 10, 12, 15, 20 minutes);
10000 g -30000 g (e.g., 10000, 15000, 20000, 25000, 30000 g) for 20 minutes -40 minutes (e.g., 20, 25, 28, 30, 32, 35, 40 minutes).

In one embodiment of the present disclosure, a first round of centrifugation is performed at 2000 g for 10 minutes.

In one embodiment of the present disclosure, a second round of centrifugation is performed at 20000 g for 30 minutes.

Specifically, parameters of the ultracentrifugation in step (4) comprise 100000 g -180000 g (e.g., 100000, 120000, 140000, 150000, 160000, 180000) for 50 minutes -100 minutes (e.g., 50, 60, 65, 70, 75, 80, 90, 100 minutes).

In one embodiment of the present disclosure, the ultracentrifugation is performed at 150000 g for 70 minutes.

Specifically, a solvent used for the resuspending in step (5) is any buffer suitable for cell culture, such as PBS, a Tris buffer, or a glycine buffer. In some embodiments of the present disclosure, the solvent used for the resuspending is PBS.

Specifically, one or more of the ultrasonic treatment, centrifugation, and ultracentrifugation are performed at low temperatures, e.g., 0°C -5°C; particularly, the ultrasonic treatment, centrifugation, and ultracentrifugation are all performed on ice.

In some embodiments of the present disclosure, the method comprises: taking a cell suspension with a density of 1×10⁶ cells/mL, placing an ultrasonic probe at a center of a liquid surface of the cell suspension, performing ultrasonic treatment with an amplitude of 20% and a duration of 15 s with a cycle of 2 s on and 2 s off; transferring a resulting liquid to a centrifuge tube for centrifugation at parameters of 2000 g×10 min, 20000 g×30 min, and collecting a resulting supernatant; and transferring the supernatant to an ultracentrifuge tube for ultracentrifugation at parameters of 150000 g×70 min.

In a third aspect of the present disclosure, use of the vesicle according to the first aspect as a drug carrier in the preparation of a drug for preventing and/or treating a disease is provided.

In a preferred embodiment of the present disclosure, the disease is an ocular disease, particularly a uveal or retinal related disease, including but not limited to uveitis, such as autoimmune uveitis; a retinal vascular disease, such as retinal artery occlusion, retinal vein occlusion, retinal periphlebitis, diabetic retinopathy, hypertensive retinopathy, retinopathy of prematurity, etc.; a macular disease, such as central serous chorioretinopathy, age-related macular degeneration, myopic macular degeneration, cystoid macular edema, macular hole, epiretinal membrane, etc.; a retinal detachment disease, such as rhegmatogenous retinal detachment, tractional retinal detachment, exudative retinal detachment, etc.; retinitis pigmentosa; a retinal tumor disease, such as retinoblastoma, etc.; and other ocular diseases, such as glaucoma, dry eye syndrome, other ocular inflammatory diseases (e.g., blepharitis, blepharokeratoconjunctivitis, conjunctivitis, keratitis, optic neuritis, etc.), optic nerve injury (e.g., optic neuritis, optic atrophy, papilledema, ischemic optic neuropathy, etc.), ocular tumors, etc.

Specifically, the ocular tumors comprise tumors of any ocular tissue or any ocular cell, including but not limited to choroidal tumors, conjunctival tumors, eyelid tumors, infiltrative intraocular tumors, iris tumors, metastatic ocular tumors, optic nerve tumors, orbital tumors, and retinal tumors. More specifically, the ocular tumors include but are not limited to choroidal tumors, such as choroidal hemangioma, choroidal melanoma, choroidal metastasis, choroidal nevus, choroidal osteoma, ciliary melanoma, and Ota's nevus; conjunctival tumors, such as conjunctival Kaposi's sarcoma, dermoid cysts on ocular surface, conjunctival lymphoma, atypical melanoma and PAM, pigmented conjunctival tumors, pinguecula, pterygium, squamous cell carcinoma, and conjunctival intraepithelial neoplasia; eyelid tumors, such as basal cell carcinoma, capillary hemangioma, hidrocystoma, nevus of the eyelid margin, seborrheic keratosis, eyelid malignant melanoma, sebaceous gland carcinoma of the eyelid, and squamous cell carcinoma of the eyelid; infiltrative intraocular tumors, such as chronic lymphocytic leukemia, eyelid tumors, choroidal and intraocular lymphoma; infiltrative choroidopathy and intraocular lymphoma; iris tumors, such as anterior uveal metastases, iris cysts, iris melanocytoma, iris melanoma, and iris pearl cysts; metastatic ocular tumors, such as metastatic choroidal melanoma; optic nerve tumors, such as choroidal melanoma affecting the optic nerve, peripapillary metastasis of optic neuropathy, optic nerve melanocytoma, and optic nerve sheath meningioma; orbital tumors, such as adenoid cystic carcinoma of the lacrimal gland, orbital cavernous hemangioma, orbital lymphangioma, orbital mucocele, orbital inflammatory pseudotumor, orbital rhabdomyosarcoma, periorbital hemangioma in childhood, and sclerosing inflammatory pseudotumor; retinal tumors, such as retinal pigment epithelium (RPE) hyperplasia, retinal pigment epithelium (RPE) tumors, retinoblastoma, and von Hippel hemangioma.

In some embodiments of the present disclosure, the vesicle is an intracellular nanovesicle modified by A25-Lamp2b overexpression (sIVs-A25), and the disease is autoimmune uveitis.

In some embodiments of the present disclosure, the vesicle is an intracellular nanovesicle modified by RGD-Lamp2b overexpression (sIVs-RGD), and the disease is a retinal neovascular disease, such as diabetic retinopathy, diabetic macular edema, retinal vascular occlusion, retinopathy of prematurity.

Specifically, the vesicle serves as a drug carrier to load a pharmaceutically active ingredient, such as a pharmaceutically active ingredient for the aforementioned ocular diseases; the pharmaceutically active ingredient comprises one or more of chemical drugs (e.g., rapamycin, methotrexate), biological drugs (e.g., protein drugs (such as PEDF, Lucentis), polypeptide drugs (such as KV11, PEDF34mer, PEDF P5-3), oligonucleotide drugs (such as siRNA)), natural drugs, nanodrugs, radiopharmaceuticals, photothermal therapy and photodynamic therapy drugs.

Specifically, the polypeptide and protein drugs may be, for example, cytokines (such as interleukins (e.g., IL-1, IL-2, IL-6, IL-10, IL-11), colony-stimulating factors (e.g., granulocyte colony-stimulating factors (G-CSFs) and macrophage colony-stimulating factors (M-CSFs)), interferons (e.g., α, β, γ interferons), growth factors (e.g., pigment epithelium-derived factors), tumor necrosis factors (e.g., TNF-α and TNF-β), transforming growth factor-β family or chemokine family, etc.), human hemoglobin, coagulation factors, anti-VEGF antibodies, protein hormones (e.g., insulin, glucagon, calcitonin, hypothalamic hormones, pituitary hormones, or gastrointestinal hormones, etc.), antibodies (e.g., monoclonal antibodies, polyclonal antibodies, dimers, multimers, multispecific antibodies, or antibody fragments), enzyme and coenzyme drugs (phenylalanine ammonia-lyase, arginase, arginine deacylase, pancreatic ribonuclease, superoxide dismutase, asparaginase, glucocerebrosidase, or hyaluronidase), other polypeptide drugs (e.g., KV11, PEDF34mer, PEDF P5-3, etc.).

In some embodiments of the present disclosure, the vesicle is sIVs-A25, and the pharmaceutically active ingredient may be a hormone, an immunosuppressive drug (rapamycin, methotrexate), an siRNA, a protein (such as IL-10), and a polypeptide.

In some embodiments of the present disclosure, the vesicle is sIVs-RGD, and the pharmaceutically active ingredient may be an siRNA, a protein (such as PEDF, Lucentis), a polypeptide (such as KV11, PEDF34mer, PEDF P5-3).

Specifically, a method for drug loading may be one or a combination of several methods selected from the group consisting of genetic engineering method, chemical synthesis method, viral vector method, ultrasonic method, electroporation method, and co-incubation method.

Specifically, the drug may be administered via any suitable administration route, particularly intraocular administration, such as eye drop administration, eye ointment administration, retrobulbar injection administration, peribulbar injection administration, subconjunctival injection administration, intravitreal injection administration, particularly intravitreal injection administration.

Specifically, the drug may be formulated into any suitable dosage form, such as, but not limited to cream, foam, paste, ointment, emulsion, liquid solution, eye drop, injection, lyophilized powder for injection, gel, spray, suspension, microemulsion, eye patch, or contact lens, etc., particularly injection.

In a fourth aspect of the present disclosure, a method for preparing a drug-loaded vesicle is provided, which comprises using the vesicle according to the first aspect of the present disclosure as a drug carrier for drug loading.

Specifically, a method for drug loading may be one or a combination of several methods selected from the group consisting of genetic engineering method, chemical synthesis method, viral vector method, ultrasonic method, electroporation method, and co-incubation method, particularly the co-incubation method.

Specifically, steps for drug loading comprise: mixing the vesicle with a pharmaceutically active ingredient, and performing incubation on a resulting mixture;
optionally, removing a free pharmaceutically active ingredient (e.g., by ultrafiltration centrifugation).

Specifically, the incubation is performed at a temperature of 35°C-40°C (e.g., 35, 36, 37, 38, 39, 40°C), particularly 37°C.

Specifically, the incubation is performed for 1-2 hours (e.g., 1, 1.2, 1.5, 2 hours), particularly 1 hour.

In some embodiments of the present disclosure, parameter of an ultrafiltration membrane is 100 kD.

Specifically, the method for preparing the drug-loaded vesicle may further comprise the steps of the method for preparing the vesicle according to the first aspect.

In a fifth aspect of the present disclosure, a drug-loaded vesicle is provided, which comprises a drug carrier, wherein the drug carrier is the vesicle according to the first aspect of the present disclosure.

Specifically, a drug in the drug-loaded vesicle is as described in the third aspect of the present disclosure.

Specifically, a drug loading rate of the drug-loaded vesicle may be 40%-90% (e.g., 45%, 50%, 55%, 60%, 65%, 70%, 80%, 90%).

Specifically, an encapsulation efficiency of the drug-loaded vesicle may be 40%-90% (e.g., 45%, 50%, 55%, 60%, 65%, 70%, 80%, 90%).

Specifically, the method for preparing the drug-loaded vesicle may be as described in the fourth aspect of the present disclosure.

In a sixth aspect of the present disclosure, a method for preventing and/or treating a disease is provided, comprising a step of administering the vesicle according to the first aspect loaded with a drug (e.g., the drug-loaded vesicle according to the fourth aspect of the present disclosure) to a subject in need thereof.

In particular, the disease is preferably an ocular disease, as described in the third aspect of the present disclosure.

Specifically, the subject is a mammal, particularly a human.

Specifically, the vesicle serves as a drug carrier to load a pharmaceutically active ingredient, such as a pharmaceutically active ingredient for the aforementioned ocular diseases; the pharmaceutically active ingredient comprises one or more of chemical drugs, biological drugs, natural drugs, nanodrugs, radiopharmaceuticals, photothermal therapy and photodynamic therapy drugs.

Specifically, the administration may be performed via any suitable administration route, particularly intraocular administration or intravenous injection; the intraocular administration comprises eye drop administration, eye ointment administration, retrobulbar injection administration, peribulbar injection administration, subconjunctival injection administration, intravitreal injection administration, particularly intravitreal injection administration. In some embodiments of the present disclosure, intravenous injection is used to treat autoimmune uveitis, and intravitreal administration is used to treat retinal neovascular diseases.

The present disclosure provides a method for preparing intracellular-derived targeted nanovesicles and uses thereof. The inventors prepare intracellular nanovesicles modified by Lamp2b-RGD overexpression (sIVs-RGD), and intracellular nanovesicles modified by A25-Lamp2b overexpression (sIVs-A25), thereby endowing the originally non-targeting vesicles with the ability to target neovascularization and T cells, respectively. Furthermore, the sIVs-RGD and sIVs-A25 with targeting ability are used as carriers to transport drugs to therapeutic targets for treating autoimmune uveitis and retinal neovascular diseases. The intracellular nanovesicles of the present disclosure possess high yield, strong targeting ability, small particle size, narrow particle size distribution range, exhibit higher encapsulation efficiency and drug loading rate when used as carriers to load drugs, and demonstrate a broader distribution range and extent via the intravitreal injection administration. The modified vesicles obtained in the present disclosure can both enhance their own therapeutic effects and serve as drug carriers, presenting excellent application prospects and research value.

### BRIEF DESCRIPTION OF DRAWINGS

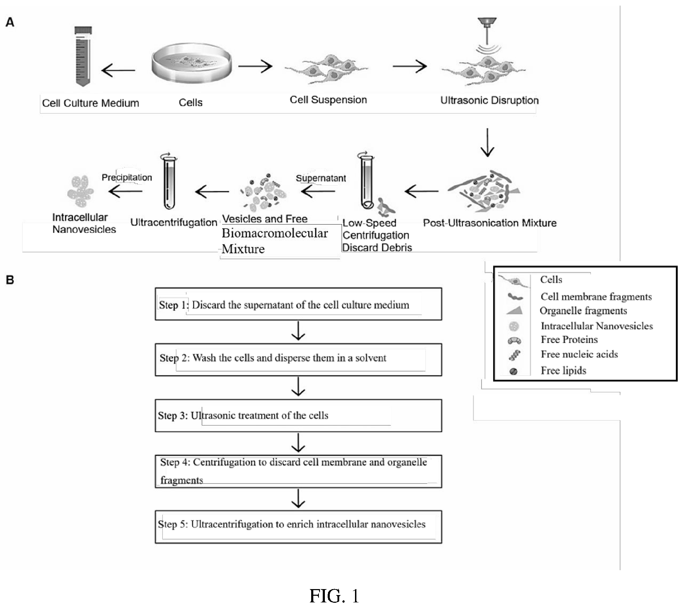

FIG. 1 illustrates a schematic diagram of the process and steps of the method for preparing intracellular nanovesicles; wherein Panel A shows the process, and Panel B shows the steps.

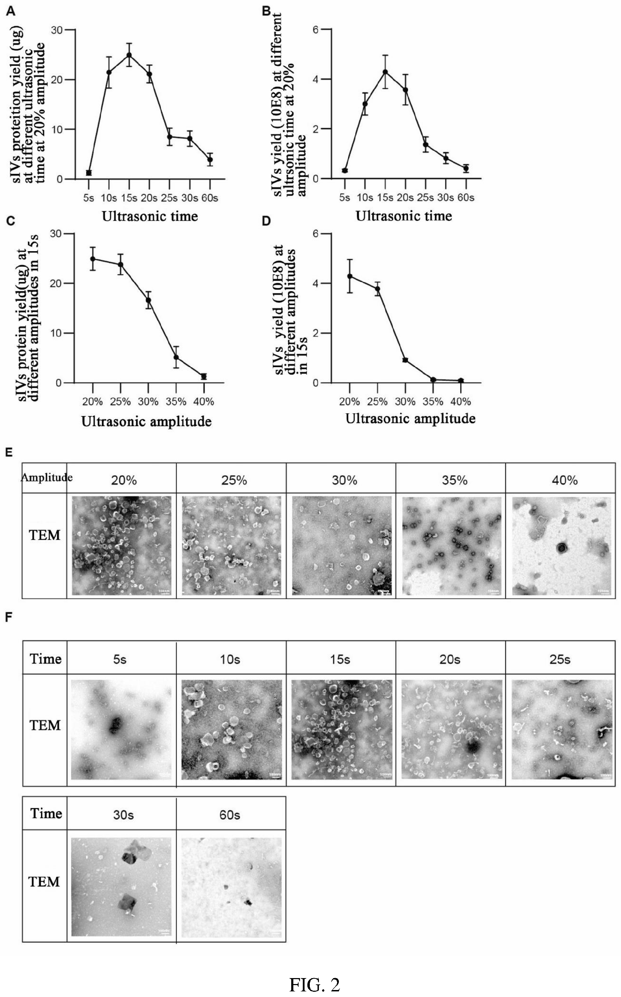

FIG. 2 illustrates an optimization process of the separation parameters for the intracellular nanovesicles; wherein Panel A and Panel B respectively show the protein yield (Panel A) and vesicle yield (Panel B) of sIVs obtained at 20% ultrasonic amplitude under different ultrasonic times; at 20% ultrasonic amplitude, when the ultrasonic time is below 10 seconds or above 20 seconds, the vesicle yield sharply decreases; Panel C and Panel D respectively show the protein yield (Panel C) and vesicle yield (Panel D) of sIVs obtained at 15s ultrasonic time under different ultrasonic amplitudes; at 15s ultrasonic time, when the ultrasonic amplitude exceeds 25%, the vesicle yield sharply decreases; Panel E shows the transmission electron micrographs of sIVs obtained at 15s ultrasonic time under different ultrasonic amplitudes, scale bar: 100 nm; Panel F shows the transmission electron micrographs of sIVs obtained at 20% ultrasonic amplitude under different ultrasonic times, scale bar: 100 nm.

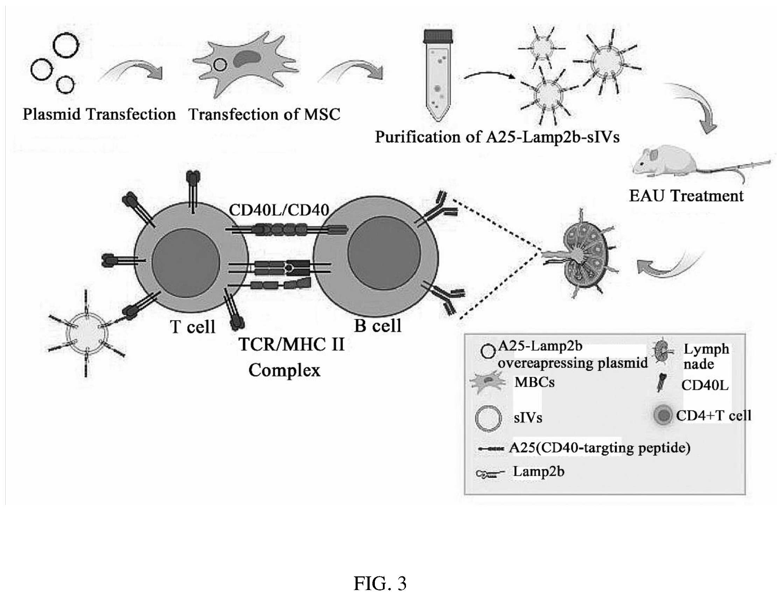

FIG. 3 shows a simplified experimental process diagram.

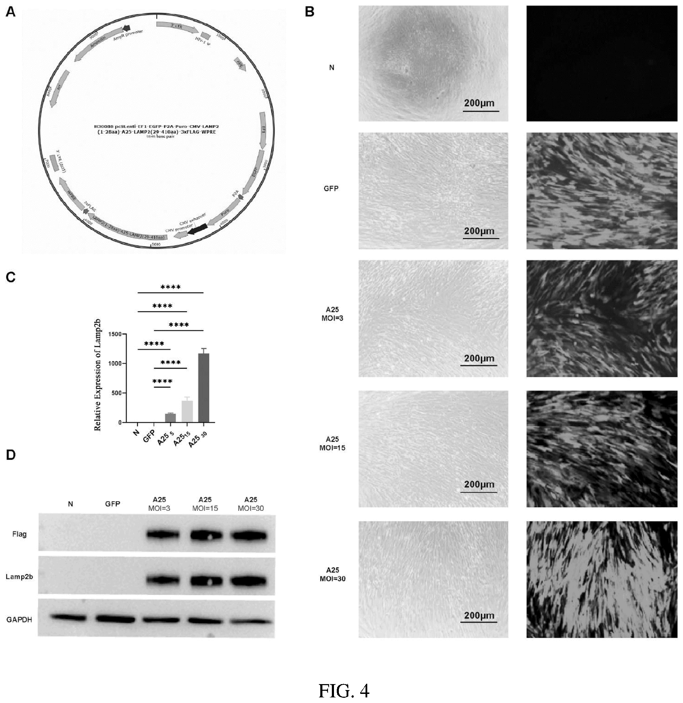

FIG. 4 shows the construction and identification of MSCs modified by A25-Lamp2b overexpression; wherein Panel A shows the A25-Lamp2b overexpression plasmid; Panel B shows the expression of green fluorescent protein (GFP) 48 h after transfecting MSCs with lentivirus at multiplicity of infection (MOI) gradients (3, 15, 30), scale bar 200 µm; Panel C shows Lamp2b mRNA expression in MSCs before and after lentiviral transfection detected by qRT-PCR; Panel D shows expression of Flag-tagged protein and Lamp2b protein in MSCs before and after lentiviral transfection detected by WB; wherein the GFP group represents MSCs transfected with empty vector virus, the A25 group represents MSCs transfected with A25-Lamp2b overexpression virus, and N represents MSCs without virus transfection.

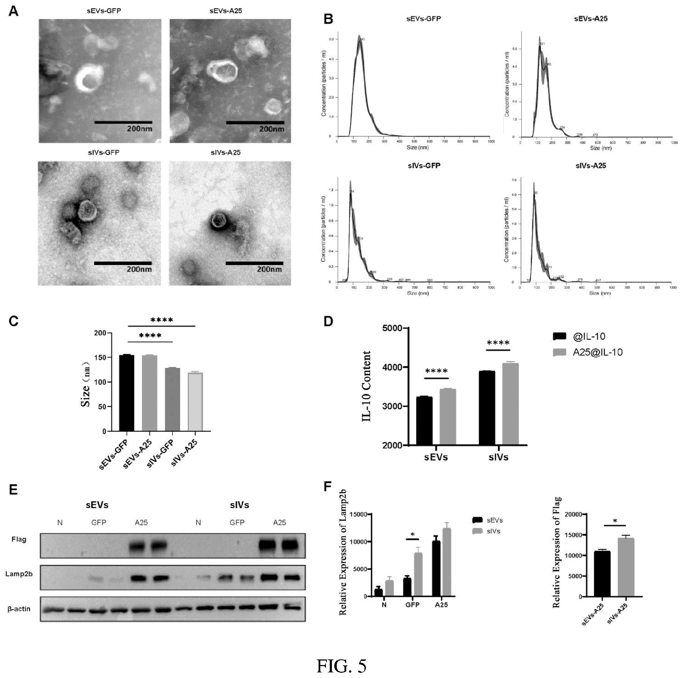

FIG. 5 shows the acquisition and identification of sEVs/sIVs-GFP/A25; Panel A shows the transmission electron micrographs of sEVs/sIVs-GFP/A25, wide-field scale bar: 200 nm, close-up scale bar: 200 nm; Panel B and Panel C show the nanoparticle size analysis results, displaying the particle size distribution of sEVs/sIVs-A25 and their statistical analysis results; Panel D shows the IL-10 content in sEVs/sIVs-A25 after loaded with IL-10; Panel E and Panel F show expression of Flag-tagged protein and Lamp2b protein in sEVs/sIVs-A25 detected by WB and their statistical analysis results (*p < 0.05, and ****p < 0.0001 indicate significant differences between groups).

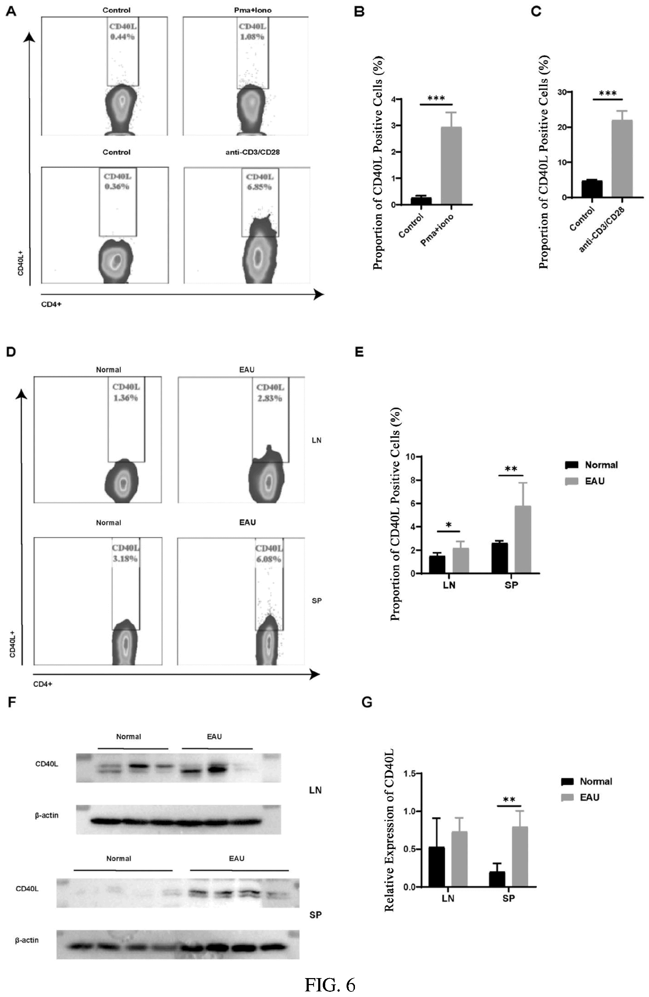

FIG. 6 shows the changes in CD40L expression in an *in vitro* T cell model and an experimental autoimmune uveitis animal model; Panel A, Panel B, and Panel C show the differences in CD40L expression detected by flow cytometry in Naive CD4⁺ T cells after 12 hours of *in vitro* stimulation and their statistical analysis results; wherein the Control group consists of CD4⁺ T cells without stimulation, the Pma+iono group consists of CD4⁺ T cells stimulated with PMA and Ionomycin, and the anti-CD3/CD28 group consists of CD4⁺ T cells stimulated with anti-CD3 and anti-CD28 antibodies. Panel D and Panel E show the differences in CD40L expression in draining lymph nodes and spleen on day 13 after EAU induction detected by flow cytometry and their statistical analysis results; wherein the Normal group consists of mice without any treatment, and the EAU group consists of mice after EAU induction. Panel F and Panel G show the differences in CD40L expression in lymph nodes and spleen on day 13 after EAU induction detected by WB and their statistical analysis results (*p < 0.05, **p < 0.01, ***p < 0.001 indicate significant differences between groups).

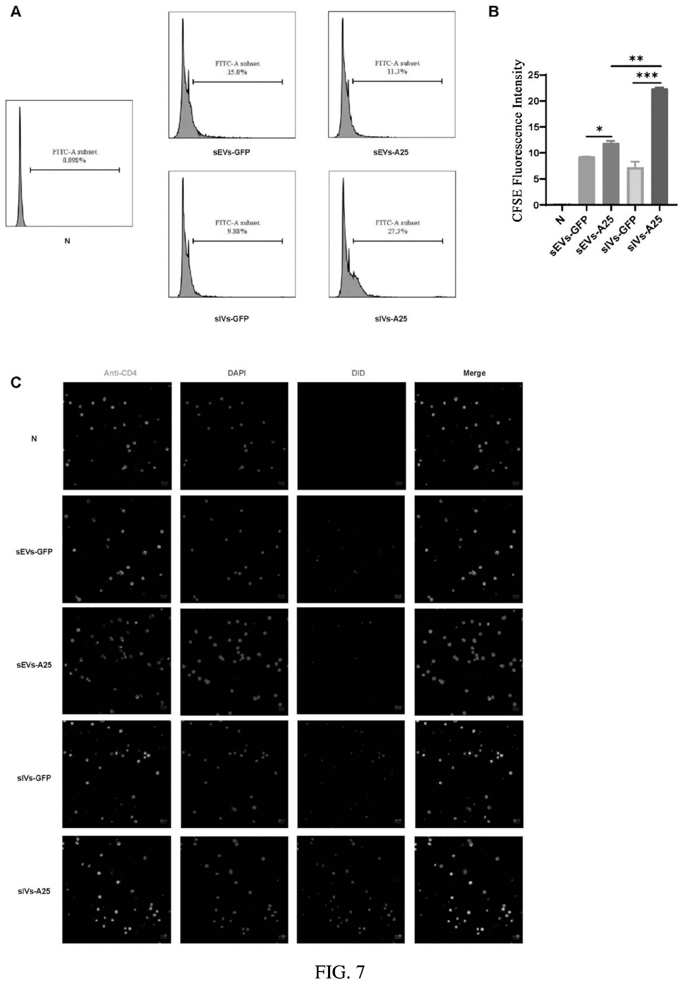

FIG. 7 shows the *in vitro* T cell targeting validation of sEVs/sIVs-A25; Panel A and Panel B show the CFSE fluorescence intensity in T cells of each group after 12 hours of co-culture detected by flow cytometry and their statistical analysis results (*p < 0.05, ***p < 0.001 indicate significant differences between groups); Panel C shows the distribution of DiD fluorescence in T cells of each group after 12 hours of co-culture detected by confocal microscopy; wherein from left to right, the high fluorescence in the first column represents CD4 expressed on the cell membrane, the high fluorescence in the second column represents DAPI, indicating the nuclei, and the high fluorescence in the third column represents DiD, indicating DiD-labeled sEVs and sIVs.

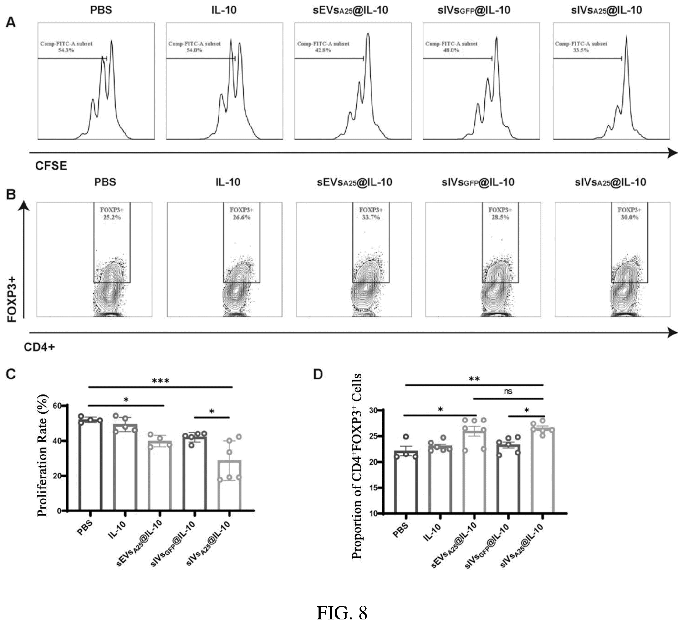

FIG. 8 shows the effect of sEVs/sIVs_{A25}@IL-10 on naive CD4⁺ T cells; Panel A and Panel C show the inhibitory effect of each sEVs and sIVs group on T cell proliferation and their statistical analysis; Panel B and Panel D show the promoting effect of each sEVs and sIVs group on Treg cell differentiation and their statistical analysis (*p < 0.05, **p < 0.01, ***p < 0.001 indicate significant differences between groups).

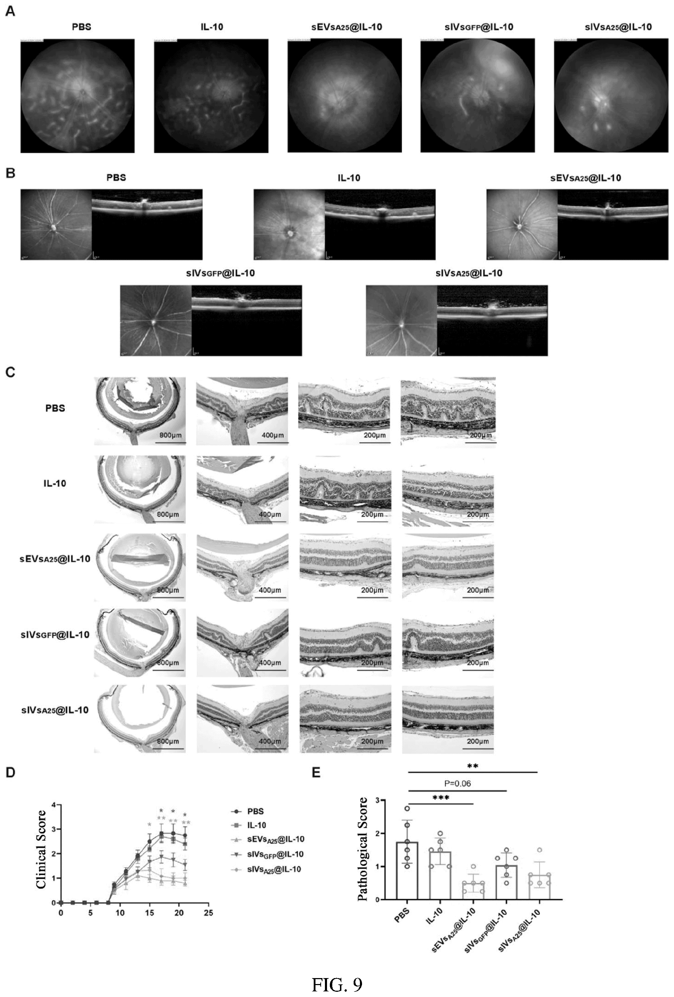

FIG. 9 shows the evaluation of fundus inflammation in EAU mice from each group; Panel A shows the fundus photographs of mice from each treatment group and the blank control group on day 17 after EAU model induction; Panel B shows the OCT images of mice from each group on day 17 after EAU model induction; Panel C shows the HE staining sections of the retinas from mice in each treatment group and the blank control group on day 21 after EAU model induction; Panel D, and Panel E show the clinical and pathological scores of mice from each treatment group and the blank control group after treatment, respectively (*p < 0.05, **p < 0.01, ***p < 0.001 indicate significant differences between groups).

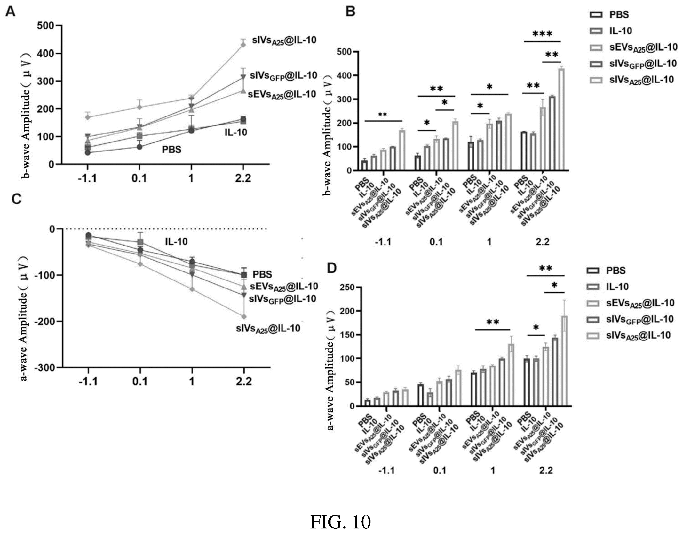

FIG. 10 shows the electroretinography (ERG) measurement results of EAU mice from each group; Panel A and Panel B show the b-wave amplitude and their statistical analysis; Panel C and Panel D show the a-wave amplitude and their statistical analysis (*p < 0.05, **p < 0.01, ***p < 0.001 indicate significant differences between groups).

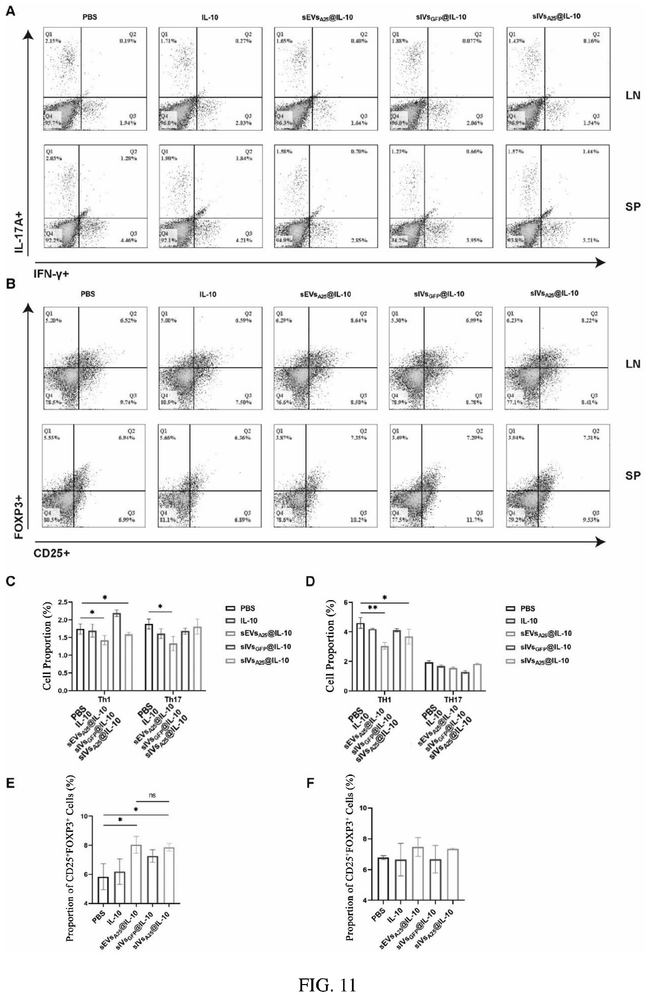

FIG. 11 shows the changes in the proportions of cell subsets in the spleens and draining lymph nodes of EAU mice from each group; Panel A shows the flow cytometry results of IL-17A⁺ cells and IFN-γ⁺ cells in the draining lymph nodes and spleens from each treatment group and the blank control group (from left to right: PBS, IL-10, sEVs_{A25}@IL-10, sIVs_{GFP}@IL-10, and sIVs_{A25}@IL-10); Panel B shows the flow cytometry results of CD25⁺FOXP3⁺ cells in the draining lymph nodes and spleens from each treatment group and the blank control group (from left to right: PBS, IL-10, sEVs_{A25}@IL-10, sIVs_{GFP}@IL-10, and sIVs_{A25}@IL-10); Panel C shows the statistical analysis results of the proportions of Th1 (IFN-γ⁺) and Th17 (IL-17A⁺) cells in the draining lymph nodes from each treatment group and the blank control group; Panel D shows the statistical analysis results of the proportions of Th1 (IFN-γ⁺) and Th17 (IL-17A⁺) cells in the spleens from each treatment group and the blank control group; Panel E shows the statistical analysis results of the proportion of Treg (CD25⁺FOXP3⁺) cells in the draining lymph nodes from each treatment group and the blank control group; Panel F shows the statistical analysis results of the proportion of Treg (CD25⁺FOXP3⁺) cells in the spleens from each treatment group and the blank control group (*p < 0.05, **p < 0.01, ***p < 0.001 indicate significant differences between groups).

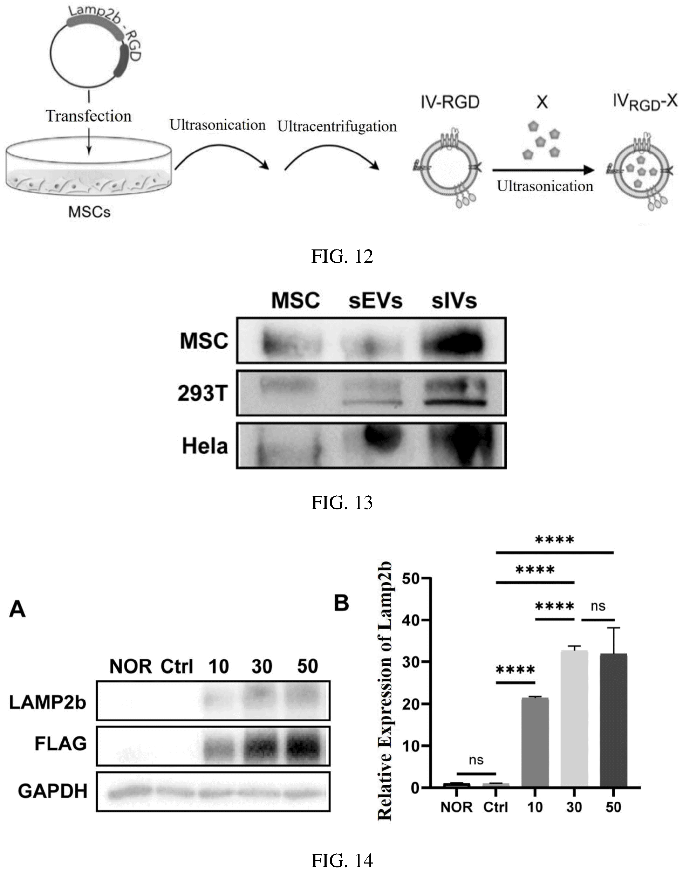

FIG. 12 shows a simplified experimental process diagram.
FIG. 13 shows the Western Blot results, displaying Lamp2b protein expression in the proteins, sIVs and sEVs of three cell types (MSCs, 293 cells, and HeLa cells).
FIG. 14 shows the Western Blot results, displaying expression of Lamp2b and Flag proteins(Panel A) and the statistical analysis results of Lamp2b expression (Panel B); GAPDH is used as the internal control; NOR indicates the group without virus transfection, Ctrl indicates the empty lentivirus transfection group, and 10/30/50 indicates the lentivirus transfection groups (where 10/30/50 represents the multiplicity of infection, MOI).

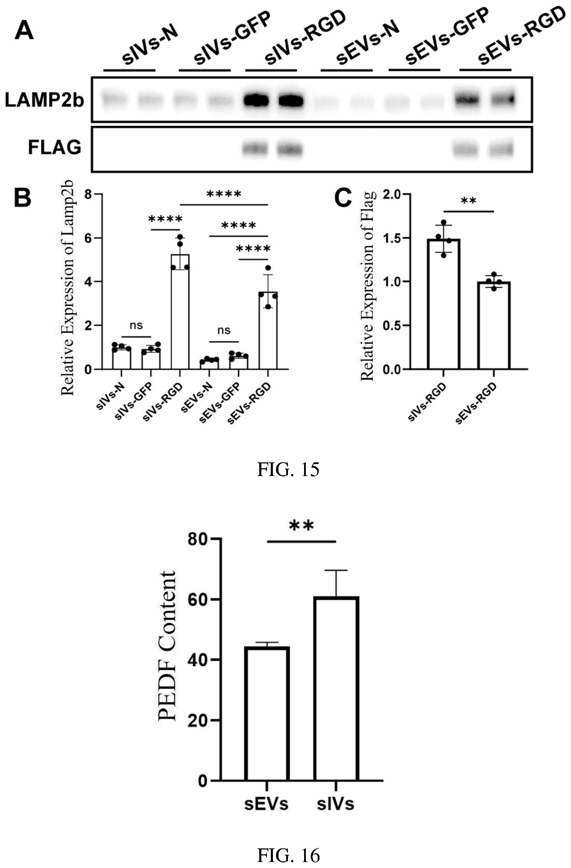

FIG. 15 shows the Western Blot results, displaying expression of Lamp2b and Flag proteins (Panel A) and the statistical analysis results (Panel B, and Panel C); GAPDH is used as the internal control; NOR indicates the group without virus transfection, Ctrl indicates the empty lentivirus transfection group, and 10/30/50 indicates the lentivirus transfection groups, where 10/30/50 represents the multiplicity of infection (MOI) (**p < 0.01, ***p < 0.001, ****p < 0.0001 indicate significant differences between groups).
FIG. 16 shows the ELISA detection results; sEVs_{RGD}@PEDF indicates the composite formulation prepared by loading sEVs with PEDF, and sIVs_{RGD}@PEDF indicates the composite formulation prepared by loading sIVs with PEDF (**p < 0.01, ***p < 0.001, and ****p < 0.0001 indicate significant differences between groups).

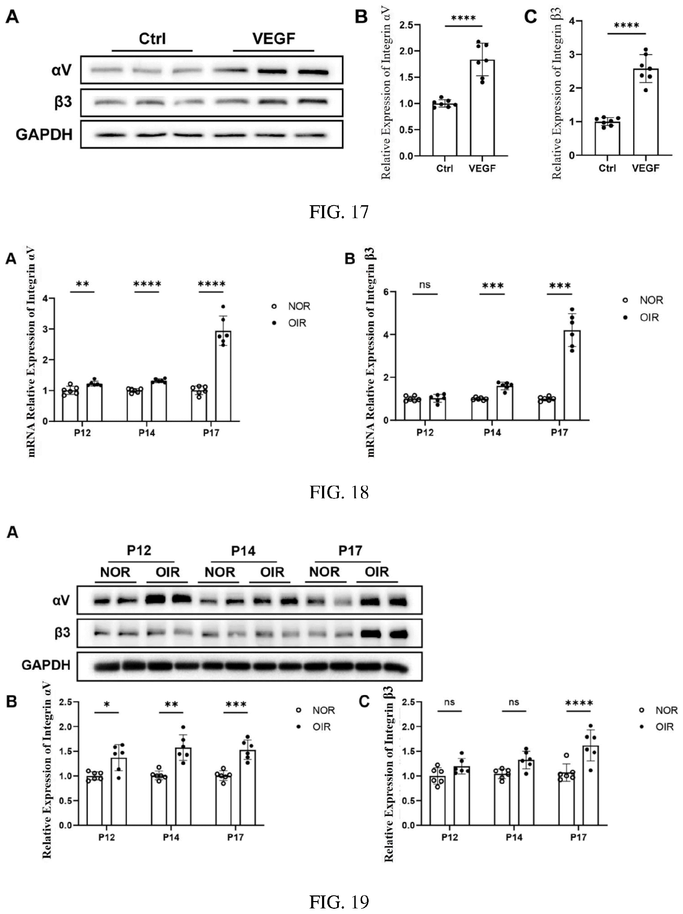

FIG. 17 shows the Western Blot results, displaying the expression of RGD-bound integrins αV and β3 in normal endothelial cells and VEGF-induced proliferative endothelial cells (Panel A) and the statistical analysis results (Panel B and Panel C) (**p < 0.01, ***p < 0.001, ****p < 0.0001 indicate significant differences between groups).
FIG. 18 shows the PCR results, displaying the expression of RGD-bound integrin αV (Panel A) and β3 (Panel B) in the retinas of normal mice and oxygen-induced retinopathy (OIR) mice (**p < 0.01, ***p < 0.001, ****p < 0.0001 indicate significant differences between groups).
FIG. 19 shows the Western Blot results, displaying the expression of RGD-bound integrins αV and β3 in the retinas of normal mice and OIR mice (Panel A) and their statistical analysis results (Panel B and Panel C) (**p < 0.01, ***p < 0.001, ****p < 0.0001 indicate significant differences between groups).

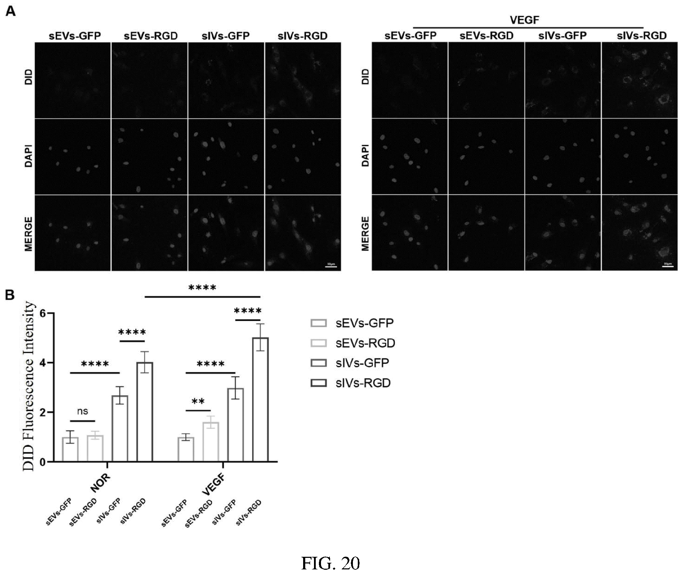

FIG. 20 shows the experimental results of vesicle uptake by endothelial cells, displaying the uptake of sEVs-GFP, sEVs-RGD, sIVs-GFP, and sIVs-RGD at 12 h in the normal endothelial cells and the proliferative endothelial cell model (Panel A) and the statistical analysis results (Panel B); the first row shows DiD-labeled vesicles, the second row shows DAPI-stained nuclei; scale bar: 50 µm (**p < 0.01, ***p < 0.001, ****p < 0.0001 indicate significant differences between groups).

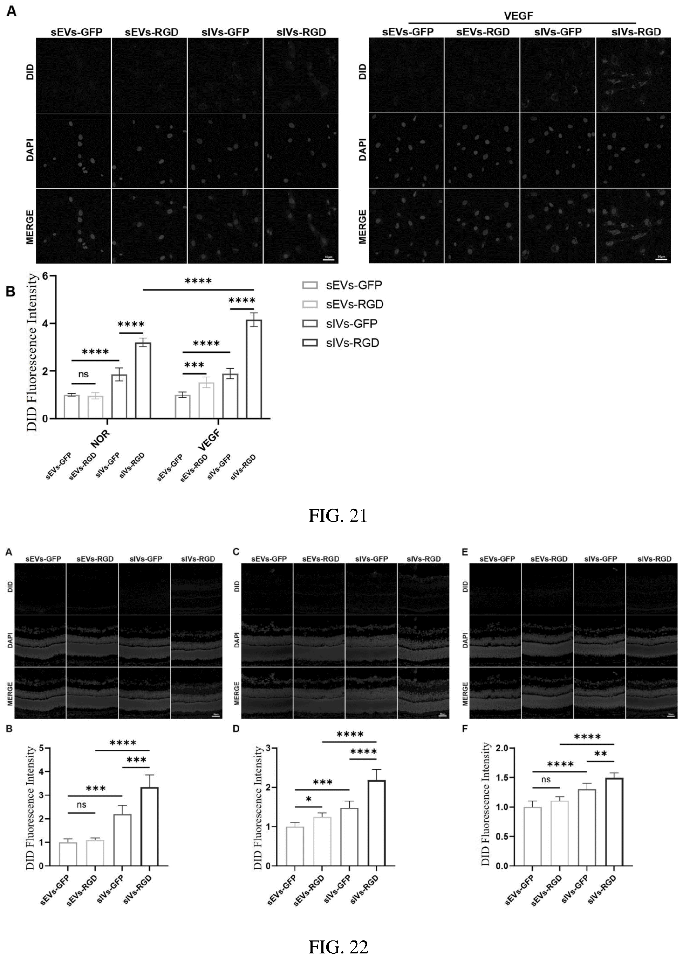

FIG. 21 shows the experimental results of vesicle uptake by endothelial cells, displaying the uptake of sEVs-GFP, sEVs-RGD, sIVs-GFP, and sIVs-RGD at 24 h in the normal endothelial cells and the proliferative endothelial cell model (Panel A) and the statistical analysis results (Panel B); the first row shows DiD-labeled vesicles, the second row shows DAPI-stained nuclei; scale bar: 50 µm (**p < 0.01, ***p < 0.001, ****p < 0.0001 indicate significant differences between groups).
FIG. 22 shows the experimental results of vesicle uptake by the retinas of OIR mice, displaying the uptake and the statistical analysis results of sEVs-GFP, sEVs-RGD, sIVs-GFP, and sIVs-RGD in the normal endothelial cells and the proliferative endothelial cell model at 8 h (Panel A and Panel B), 24 h (Panel C and Panel D), and 5 days (Panel E and Panel F) after intravitreal injection; the first row shows DiD-labeled vesicles, the second row shows DAPI-stained nuclei; scale bar: 50 µm (**p < 0.01, ***p < 0.001, ****p < 0.0001 indicate significant differences between groups).

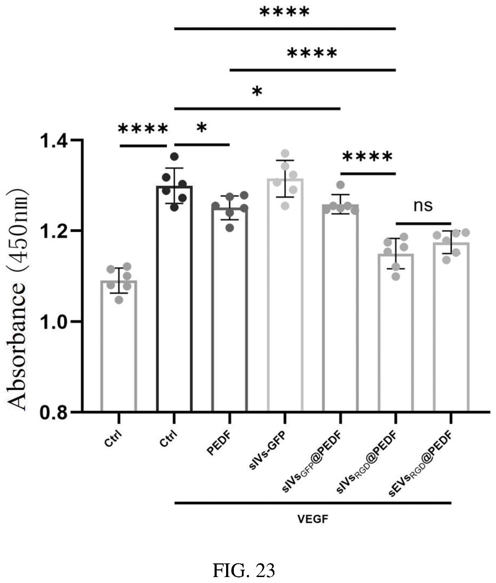

FIG. 23 shows the *in vitro* CCK assay results, displaying the therapeutic effect of sIVs_{RGD}@PEDF on cell proliferation in the VEGF-induced neovascularization model *in vitro* (**p < 0.01, ***p < 0.001, ****p < 0.0001 indicate significant differences between groups).

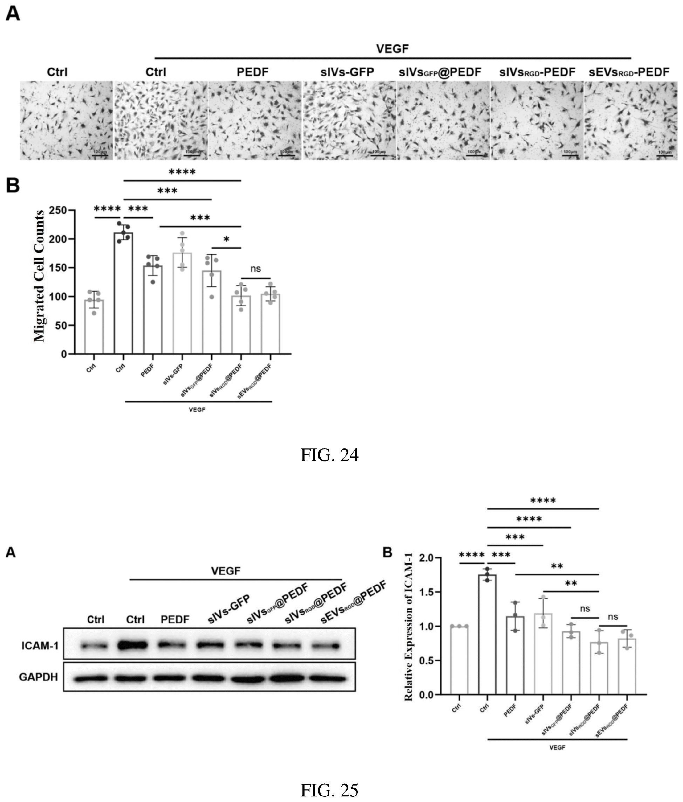

FIG. 24 shows the *in vitro* Transwell assay results, displaying the microscopic examination results (Panel A) and statistical results of specific migrated cell counts (Panel B) for the therapeutic effect of sIVs_{RGD}@PEDF on cell migration in the VEGF-induced neovascularization model *in vitro*; scale bar: 100 µm (**p < 0.01, ***p < 0.001, ****p < 0.0001 indicate significant differences between groups).
FIG. 25 shows the Western Blot results, displaying the expression of the inflammatory factor ICAM-1 in HRECs (Panel A) and the statistical analysis results (Panel B) (**p < 0.01, ***p < 0.001, ****p < 0.0001 indicate significant differences between groups).

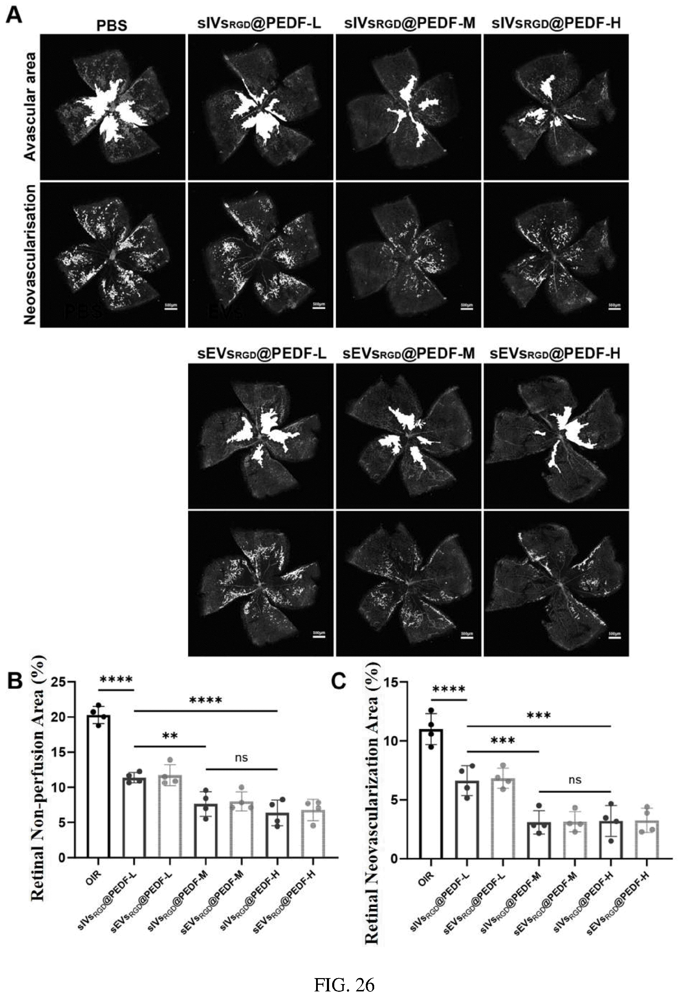

FIG. 26 shows the retinal flat-mount staining results of OIR mice, displaying the non-perfusion area and neovascularization area in the retinas of OIR mice (Panel A), the statistical analysis results of the non-perfusion area (Panel B), and the statistical analysis results of the neovascularization area (Panel C); L represents low concentration (0.5 mg/mL), M represents medium concentration (1 mg/mL), H represents high concentration (2 mg/mL) (**p < 0.01, ***p < 0.001, ****p < 0.0001 indicate significant differences between groups).

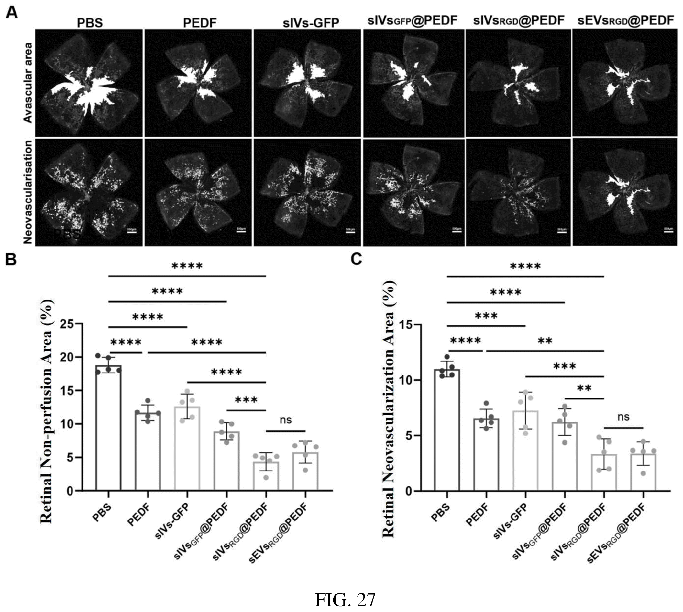

FIG. 27 shows the retinal flat-mount staining results of OIR mice, displaying the non-perfusion area and neovascularization area in the retinas of OIR mice (Panel A), the statistical analysis results of the non-perfusion area (Panel B), and the statistical analysis results of the neovascularization area (Panel C) (**p < 0.01, ***p < 0.001, ****p < 0.0001 indicate significant differences between groups).

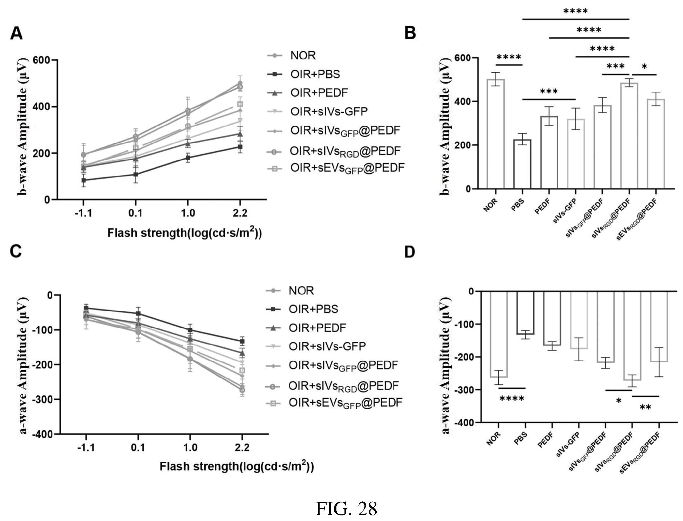

FIG. 28 shows the ERG measurement results of OIR mice from each group, displaying the overall b-wave amplitude (Panel A) and the statistical analysis of b-wave amplitude at the highest stimulus (2.2 cd•s/m²) (Panel B), the overall a-wave amplitude (Panel C) and the statistical analysis of a-wave amplitude at the highest stimulus (2.2 cd•s/m²) (Panel D) (*p < 0.05, **p < 0.01, ***p < 0.001 indicate significant differences between groups).

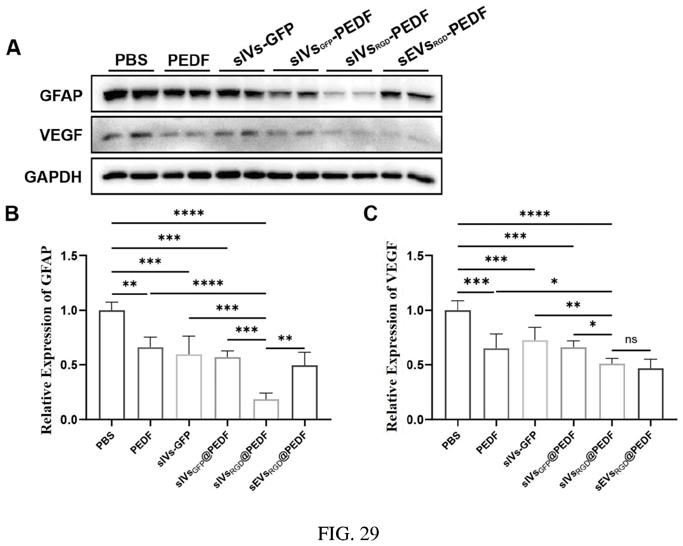

FIG. 29 shows the Western Blot results, displaying the expression of GFAP and VEGF in the retinas of mice (Panel A), the statistical analysis results of GFAP (Panel B), and the statistical analysis results of VEGF (Panel C) (**p < 0.01, ***p < 0.001, ****p < 0.0001 indicate significant differences between groups).

### DETAILED DESCRIPTION

Unless otherwise defined, all scientific and technical terms used in the present disclosure have the same meanings as commonly understood by those skilled in the art to which the present disclosure pertains.

The terms "patient" or "subject" and the like are used interchangeably herein to refer to any animal or cell thereof, whether *in vitro* or *in situ*, to be treated according to the methods described herein. Specifically, the aforementioned animals include mammals, for example, rats, mice, guinea pigs, rabbits, dogs, monkeys, humans, particularly humans.

The term "treating" refers to preventing, curing, reversing, attenuating, alleviating, minimizing, suppressing, halting, and/or stopping one or more clinical symptoms of a disease after the onset of the disease.

The term "preventing" refers to treating before the onset of a disease to avoid, minimize, or make it difficult for the disease to occur or develop.

In the present disclosure, an intracellular nanovesicle modified by A25-Lamp2b overexpression is denoted as "sIVs-A25" or "sIVs_{A25}".

In the present disclosure, an extracellular vesicle modified by A25-Lamp2b overexpression is denoted as "sEVs-A25" or "sEVs_{A25}".

In the present disclosure, an intracellular nanovesicle modified by RGD-Lamp2b overexpression is denoted as "sIVs-RGD" or "sIVs_{RGD}".

In the present disclosure, an extracellular vesicle modified by RGD-Lamp2b overexpression is denoted as "sEVs-RGD" or "sEVs_{RGD}".

Various publications, patents, and published patent specifications cited herein are hereby incorporated by reference in their entirety.

The technical solutions of the present disclosure will be clearly and completely described below in conjunction with the embodiments of the present disclosure. It is apparent that the described embodiments are only a part of the embodiments of the present disclosure, rather than all of the embodiments. Based on the embodiments of the present disclosure, all other embodiments obtained by those of ordinary skill in the art without creative efforts shall fall within the scope of protection of the present disclosure.

### Example 1: Cell Extraction and Culture

### 1. Extraction and Culture of Mesenchymal Stem Cells (MSCs)

Human umbilical cords were provided by Beijing Beilai Biotechnology Co., Ltd, China. Umbilical cords obtained from normal pregnancy without complications after cesarean section were immediately placed in saline containing penicillin (100 U/mL) and streptomycin (100 mg/mL), and then transported to the laboratory within 4 hours. After removing residual blood and blood vessels, the obtained umbilical cords were cut into 1-3 mm pieces and digested with 0.1% type II collagenase at 37°C for 1 hour. A resulting suspension was then filtered through a 100-mesh sieve to remove undigested tissue. A resulting filtered supernatant was centrifuged and washed three times with PBS. The cell pellet was resuspended in Dulbecco's Modified Eagle Medium/Nutrient Mixture F-12 complete medium. The medium contained 10% fetal bovine serum (FBS), 100 U/mL penicillin, and 100 mg/mL streptomycin. The cells were seeded in T175 culture flasks and cultured in a 5% CO₂ incubator at 37°C. The medium was changed every 3 days. When cell confluence reached 80%, the cells were passaged at a split ratio of 1:2. Cells at passages 3 to 5 (P3-P5) were used for experiments.

### 2. Culture of 293T Cells

Cells were cultured using DMEM complete medium (DMEM, Invitrogen, USA) with 5% fetal bovine serum and 1% penicillin-streptomycin (ScienCell, Carlsbad, CA). The cells were incubated at 37 °C with 5% CO₂. The medium was changed every 2-3 days. Cells at passages 3 to 6, with 70-80% confluence, were used for various experiments.

### 3. Culture of HeLa Cells

Cells were cultured using DMEM complete medium (DMEM, Invitrogen, USA) with 5% fetal bovine serum and 1% penicillin-streptomycin (ScienCell, Carlsbad, CA). The cells were incubated at 37 °C with 5% CO₂. The medium was changed every 2-3 days. Cells at passages 3 to 6, with 70-80% confluence, were used for various experiments.

The culture of the above cells can also refer to the Chinese patent application with application number 202410605857.X.

### Example 2: Preparation of Cell-Derived Nanovesicles

The process of the method for preparing intracellular nanovesicles was shown in FIG. 1.

### 1. Cell Digestion and Counting

Taking MSCs as an example, when the cells reached 90% confluence, the supernatant was completely aspirated. PBS was added to wash the cells twice. Trypsin was then added to digest the cells. After digestion, PBS was used to neutralize and wash the cells three times. Cell counting was subsequently performed, and cell concentration was adjusted to 1×10⁶ cells/mL using PBS.

### 2. Ultrasonic Treatment of Cells

2 mL of cell suspension with a density of 1×10⁶ cells/mL was taken and added to the bottom of a 50 mL centrifuge tube. An ultrasonic probe was placed at the center of liquid surface for ultrasonic treatment. During the ultrasonic treatment, an ultrasonic amplitude parameter was set to 20%, a time parameter was set to 15s, with a cycle of 2 s on and 2 s off, and the centrifuge tube was placed on ice. Subsequently, a resulting liquid was transferred to a 2 mL centrifuge tube for centrifugation.

### 3. Collection of Small Intracellular Nanovesicles

Parameters of the centrifugation were 2000 g × 10 min, 20,000 g × 30 min. A resulting supernatant was then collected and transferred to an ultracentrifuge tube. Parameters of ultracentrifugation were 150,000 g × 70 min. All the above operations were performed on ice. An obtained pellet was resuspended in PBS to yield small intracellular nanovesicles (sIVs).

### 4. Collection of Small Extracellular Vesicles

FBS is an essential component for cell culture *in vitro*; however, it contains a large number of bovine-derived extracellular vesicles, which are also present in complete cell culture medium containing FBS. To remove bovine extracellular vesicles, FBS was centrifuged at 4°C at 110,000 × g overnight (approximately 12 hours). When cells reached 60% confluence, the cells were cultured in complete medium containing 10% exosome-depleted FBS for 48 hours. Then, supernatant was collected, and extracellular vesicles were isolated by ultracentrifugation at 4 °C. The specific steps comprised ultracentrifugation at 300 g × 10 min, 2000 g × 10 min, 10000 g × 30 min, and two rounds of 110000 g × 70 min. An obtained pellet was resuspended in PBS to yield small extracellular vesicles (sEVs) predominantly composed of exosomes.

### Example 3: Optimization of Separation Parameters for Intracellular Nanovesicles

By following the steps in Example 2, the optimization process of the separation parameters for the intracellular nanovesicles was shown in FIG. 2.

### 1. Detection of sIVs Yield Differences by Nanoparticle Tracking Analysis

PBS-resuspended sIVs were diluted to 1 mL with PBS, and detected using Nanoparticle Tracking Analysis (NTA) software NTA 3.3 Dev Build 3.3.104. Temperature was set to 25°C, laser was set to Blue488, flow rate was set to 50, mode was set to automatic detection. Sample injection was performed for three times, and analysis was performed for three times. Average peak value was taken as the Mode particle size result. Camera mode was sCMOS. Laser type was Blue488, and viscosity was 0.9 cP.

### 2. Observation of the Morphology of sIVs from Different Cells by Transmission Electron Microscopy (TEM)

The centrifuged sIVs were resuspended in 200 µL of PBS and mixed well. 10 µL of the sIVs solution was mixed with 4% PFA at a volume ratio of 1:1, and dropped on a clean plastic film to form a droplet. Then, the front side of a TEM carbon grid was placed on the droplet for 20 min. 10 µL of phosphotungstic acid was used for negative staining for 90s. The carbon grid was dried by baking, and observation was performed using a HitacW-7500 transmission electron microscope.

Panel A and Panel B of FIG. 2 respectively showed the protein yield and vesicle yield of sIVs obtained according to the steps of this example at 20% ultrasonic amplitude and ultrasonic times of 5s, 10s, 15s, 20s, 25s, 30s, and 60s. The results showed that when the ultrasonic time was below 10 seconds or above 20 seconds, the number of vesicles and protein yield sharply decreased. Panel C and Panel D of FIG. 2 respectively showed the protein yield and vesicle yield of sIVs obtained according to the steps of this example at 15s ultrasonic time and amplitudes of 20%, 25%, 30%, 35%, and 40%. The results showed that at ultrasonic time of 15s, when the ultrasonic amplitude exceeded 25%, the vesicle yield sharply decreased. Panel E of FIG. 2 showed the transmission electron micrographs of sIVs obtained according to the steps of this example at 15s ultrasonic time and ultrasonic amplitudes of 20%, 25%, 30%, 35%, and 40%. Panel F of FIG. 2 showed the transmission electron micrographs of sIVs obtained according to the steps of this example at 20% ultrasonic amplitude and ultrasonic times of 5s, 10s, 15s, 20s, 25s, 30s, and 60s.

This optimization process indicated that at ultrasonic amplitude of 20%, when the ultrasonic time was below 10 seconds or above 20 seconds, the vesicle yield sharply decreased. At ultrasonic time of 15s, when the ultrasonic amplitude exceeded 25%, the vesicle yield sharply decreased. An amplitude of 20% and an ultrasonic time of 15s were the optimal parameters for collecting intracellular nanovesicles.

For the preparation of cell vesicles, reference may be made concurrently to the Chinese patent application with application number 202410605857.X.

### Example 3: Preparation of Nanovesicle sEVs/sIVs_{A25}@IL-10 Complexes Modified by A25-Lamp2b Overexpression

The steps for constructing nanovesicle sEVs/sIVs_{A25}@IL-10 complexes modified by A25-Lamp2b overexpression and conducting *in vitro* and *in vivo* mouse experiments were shown in FIG. 3.

### 1. Experimental Instruments and Materials

### 1.1 Experimental Reagents

DMEM-F12 Culture Medium (Gibco, USA); Type II Collagenase (Gibco, USA); Phosphate Buffered Saline (PBS) (Gibco, USA); Fetal Bovine Serum (FBS) (Gibco, USA); Insulin-Transferrin-Selenium (Gibco, USA); Penicillin-Streptomycin (Gibco, USA); Trypsin (0.25% Trypsin-EDTA) (Gibco, USA); Glutamine (Gibco, USA); Polybrene (Solarbio, China); BCA (Solarbio, China); Triton X-100 (Solarbio, China); RevertAid^{™} First Strand cDNA Synthesis Kit (Thermo, USA); GAPDH, Lamp2b Primers (Genewiz, Suzhou, China); Flag-tag, GAPDH, Lamp2b Antibodies (Abcam, USA); 75 cm², 175 cm² Cell Culture Flasks (Corning, USA); -80°C Ultra-low Temperature Freezer (Thermo Fisher Scientific, USA); 10 mL, 25 mL Pipettes (Junyao, Tianjin, China).

### 1.2 Experimental Instruments

Water Bath (Tianjin Honour Instrument Co., Ltd, China); Pipettes (Eppendorf, Germany); Benchtop Centrifuge (Eppendorf, Germany); Inverted Phase Contrast Microscope (OLYMPUS, Japan); High-pressure Steam Sterilizer (SAKURA, Japan); Electronic Balance (Sartorius, Germany).

### 2. Experimental Methods:

### 2.1. Construction of Cells Modified by A25-Lamp2b Overexpression

### 2.1.1. Construction of A25-Lamp2b Fusion Protein Overexpression Plasmid

A cDNA fragment encoding the A25 peptide and Lamp2b (atggtgtgcttccgcctcttcccggttccgggctcagggctcgttctggtctgcctagtcctgggagctgtgcggtcttatgcaGAGAGCGAGGAGGAGG ACttggaacttaatttgacagattcagaaaatgccacttgcctttatgcaaaatggcagatgaatttcacagtacgctatgaaactacaaataaaacttataaaactgtaacc atttcagaccatggcactgtgacatataatggaagcatttgtggggatgatcagaatggtcccaaaatagcagtgcagttcggacctggcttttcctggattgcgaattttacc aaggcagcatctacttattcaattgacagcgtctcattttcctacaacactggtgataacacaacatttcctgatgctgaagataaaggaattcttactgttgatgaacttttggc catcagaattccattgaatgacctttttagatgcaatagtttatcaactttggaaaagaatgatgttgtccaacactactgggatgttcttgtacaagcttttgtccaaaatggcac agtgagcacaaatgagttcctgtgtgataaagacaaaacttcaacagtggcacccaccatacacaccactgtgccatctcctactacaacacctactccaaaggaaaaac cagaagctggaacctattcagttaataatggcaatgatacttgtctgctggctaccatggggctgcagctgaacatcactcaggataaggttgcttcagttattaacatcaac cccaatacaactcactccacaggcagctgccgttctcacactgctctacttagactcaatagcagcaccattaagtatctagactttgtctttgctgtgaaaaatgaaaaccga ttttatctgaaggaagtgaacatcagcatgtatttggttaatggctccgttttcagcattgcaaataacaatctcagctactgggatgcccccctgggaagttcttatatgtgcaa caaagagcagactgtttcagtgtctggagcatttcagataaatacctttgatctaagggttcagcctttcaatgtgacacaaggaaagtattctacagcccaagagtgttcgct ggatgatgacaccattctaatcccaattatagttggtgctggtctttcaggcttgattatcgttatagtgattgcttacgtaattggcagaagaaaaagttatgctggatatcaga ctctg (SEQ ID NO: 1)) was inserted downstream of the CMV promoter in a vector plasmid to assemble the A25-Lamp2b-Flag overexpression plasmid. The plasmid structure was shown in Panel A of FIG. 4.

### 2.1.2. Isolation and Purification of Lentivirus Overexpressing the A25-Lamp2b-Flag Fusion Protein

The overexpression plasmid obtained from 2.1.1 was submitted to a company for packaging corresponding hightiter A25-Lamp2b-Flag lentivirus, wherein Flag is a common fusion tag protein composed of eight amino acids (Asp-Tyr-Lys-Asp-Asp-Asp-Asp-Lys (SEQ ID NO: 4)). After its fusion expression with the target protein, it can be effectively recognized by anti-Flag antibodies, thereby facilitating the detection and identification of the Flag-containing fusion protein A25-Lamp2b by Western Blot.

### 2.1.3. Lentiviral Infection of MSCs

When P2 generation of MSCs or 293T cells reached 60% confluence, lentivirus and 5 µg/mL Polybrene were added according to the MOI (Multiplicity of Infection) with a half-volume medium change. After 8 hours of infection, fresh complete medium was replaced. Fluorescence expression was observed under a fluorescence microscope after 48 hours (Panel B of FIG. 4).

### 2.1.4. Detection of Target Gene Lamp2b Expression by RT-PCR

P5 generation of MSCs or 293T cells were collected, with normal cells not infected with lentivirus serving as the control group. Total RNA was extracted using a Trizol reagent (Invitrogen, USA). cDNA was synthesized using a reverse transcription kit (Thermo, USA) according to the kit instruction. Reverse primers, cDNA, and SYBR Green Master (Roche, Switzerland) were sequentially added to a 384-well plate for RT-PCR, with GAPDH expression used as an internal control. Relative mRNA expression levels were calculated according to the ΔΔCt method (Panel C of FIG. 4). Lamp2b amplification primers were designed based on the NCBI gene sequence database: F: TGGCTCCGTTTTCAGCATTG (SEQ ID NO: 2); R: TGGTGTCATCATCCAGCGAA (SEQ ID NO: 3).

### 2.1.5. Detection of Target Protein Lamp2b and Flag-tagged Protein Expression by Western Blot

20 µL of sample was aspirated, mixed with a protein loading buffer, denatured at 95°C in a metal bath for 10 minutes, electrophoresed on SDS-PAGE gel, and transferred to a membrane. The membrane was blocked with 5% skimmed milk powder for 2 hours, incubated overnight with primary antibody at 4°C, washed three times with TBST solution, each time for 10 minutes, incubated with secondary antibody at room temperature for 2 hours, washed three times with TBST solution, each time for 10 minutes, and developed using ECL to observe target bands (Panel D of FIG. 4).

### 2.2.1. Isolation and Storage of sIVs/sEVs

Taking MSCs as an example, after culturing in complete medium for 2 days, the medium was replaced with medium containing 10% exosome-free serum. When cell density reached 70-80%, the cells were digested with trypsin, washed twice with PBS, and a cell suspension with a concentration of 1×10⁶/mL was prepared. The cell suspension was subjected to ultrasonic treatment to disrupt cell membrane (2 mL, power: 20%-30%, 2 s/2 s, 15 s-120 s). A resulting post-ultrasonication suspension was collected, subjected to gradient centrifugations at 2000 g for 10 min followed by 20000 g for 30 min to remove cell debris, and then subjected to ultracentrifugation (150,000× g for 70 min) to obtain sIVs. sEVs were obtained according to steps 1-4 of Example 2 and similar methods. sIVs/sEVs overexpressing A25-Lamp2b were the targeted modified sIVs/sEVs, while sIVs/sEVs transfected with empty vector virus were the unmodified sIVs/sEVs, hereafter referred to as sIVs-A25, sEVs-A25, sIVs-GFP, and sEVs-GFP.

### 2.3. Preparation and Identification of sIVs_{A25}@IL-10 Complexes

### 2.3.1 Preparation of sIVs_{A25}@IL-10 Complexes

IL-10 drug solution was sequentially added to sIVs-A25 according to mass ratio gradients (1:20, 1:10, 1:5, 1:1), mixed well, and left to stand in a 37°C incubator for 1 hour. A resulting liquid was added to a 100 kDa ultrafiltration tube and centrifuged to remove free drug. The ultrafiltration tube was pre-wet with PBS, added with the liquid and centrifuged at 14,000g for 20 min, added with 300 µL of PBS and centrifuged at 14,000 g for 20 min (the step of which was repeated twice), and then inverted for centrifugation at 4000 g for 2 min to recover and obtain sIVs_{A25}@IL-10, which was temporarily stored at 4°C. Similar methods were used to obtain sIVs_{GFP}@IL-10, sEVs_{A25}@IL-10, and sEVs_{GFP}@IL-10. Among these, sIVs_{A25}@IL-10 and sEVs_{A25}@IL-10 represent the targeted modified sIVs and sEVs loaded with IL-10, while sIVs_{GFP}IL-10 and sEVs_{GFP}@IL-10 represent the unmodified sIVs and sEVs loaded with IL-10.

### 2.3.2 Identification of sIVs_{A25}@IL-10 Complexes

2.3.2.1 Electron Microscopy: After fixation with 2% paraformaldehyde, the morphology and size of sIVs_{A25}@IL-10 complexes were identified by transmission electron microscopy. Specific method: 10 µL of suspensions of small intracellular nanovesicles and small extracellular vesicles were dropped onto a copper grid and allowed to settle for 5-10 min; 10 µL of 2% uranyl acetate was aspirated and dropped onto the copper grid for staining for 1-2 min, excess liquid was removed with filter paper; the copper grid was dried at room temperature for several minutes, loaded onto a microscope, and imaged at 80 kV to observe the morphology.

2.3.2.2 Nanosight Laser Particle Size Analyzer: Small intracellular nanovesicles and small extracellular vesicles resuspended in PBS were diluted to 1 mL with PBS and detected using NTA 3.3 Dev Build 3.3.104. Temperature was set to 25°C, laser was set to Blue488, flow rate was set to 50, mode was set to automatic detection. Sample injection was performed for three times, and analysis was performed for three times. Average peak value was taken as the Mode particle size result.

### 2.3.2.3 Western Blot: The procedure was the same as 2.1.5.

### 2.3.3 Determination of IL-10 Content in sIVs_{A25}@IL-10 Complexes

ELISA technology was used to determine drug loading amounts in sIVs_{A25}@IL-10, sEVs_{A25}@IL-10, sIVs_{GFP}@IL-10, and sEVs_{GFP}@IL-10, which comprises: the nanovesicles from each group were respectively taken, added into the wells of an ELISA kit plate, added with corresponding standard control groups, and incubated at room temperature for 1 h. Corresponding volumes of primary antibodies were added and incubated at room temperature for 1 h. Then, the plate was washed three times. Corresponding volumes of secondary antibodies were added and incubated at room temperature for 1 h. Then, the plate was washed three times. Corresponding volume of substrate was added for color development, incubated at room temperature for 20 min, and then added with corresponding volume of stop solution to terminate color development. Absorbance value at 562 nm excitation was measured with a microplate reader.

### 2.4 Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results:

### 3.1 Construction and Identification of A25-Lamp2b Overexpressing MSCs

Due to the non-phagocytic nature of T cells, targeted drug delivery to T cells still faces technical challenges. In T cell targeting research, the most commonly selected receptors for modification are T cell markers, including CD3, CD4, CD7, and CD8. However, such targeting therapy directed at all T cell subsets may lead to dose-limiting toxicity. Therefore, markers for specific cell subsets or markers overexpressed under disease conditions should be preferentially selected. CD40 ligand (CD40L, CD154) is a 39 kDa type II transmembrane protein belonging to the TNF superfamily and is considered an early activation marker of CD4⁺ T lymphocytes. CD40/CD40L is involved in triggering various downstream immune signaling pathways. Early studies have proven that CD40/CD40L costimulatory signaling transduction is necessary for generating T cell-dependent antibody responses and memory B cell differentiation. Upon activation of CD40/CD40L signaling in macrophages and dendritic cells (DCs), cytokine secretion and surface activation molecule expression are induced, participating in the regulation of cross-stimulation and activation of CD4⁺ T cells and CD8⁺ T cells. During tissue inflammation, CD40/CD40L activation in epithelial or endothelial cells induces cytokine and chemokine secretion, which may trigger additional leukocyte infiltration. Dysregulated expression of CD40/CD40L signaling pathway is also observed in various autoimmune diseases. For example, CD40L expression is significantly elevated in various cell subsets of patients with systemic lupus erythematosus (SLE), including B cells, T cells, and monocytes, as well as in circulating T cells of patients with psoriatic arthritis (PsA) and rheumatoid arthritis (RA). Additionally, overexpression of CD40L in T cells of RA patients is associated with poor prognosis. Furthermore, studies have found that soluble CD40L is also increased in several autoimmune diseases. The above results indicate that dysregulation of the CD40/CD40L signaling pathway may promote the initiation or maintenance of pathogenic autoimmune responses.

Therefore, targeting the CD40/CD40L pathway holds promise as a novel therapeutic strategy for autoimmune diseases. Currently, anti-CD40L therapy has shown to be effective in SLE, experimental autoimmune encephalomyelitis (EAE), and EAU mouse models. Clinical trials of anti-CD40L therapy have also been conducted. Early results indicated that although the drugs had clinical efficacy, they were all terminated due to the occurrence of thromboembolism. Thrombosis may be related to the binding of the Fc domain of CD40L monoclonal antibody to FcγRIIa (CD32a) on the surface of human platelets. Therefore, ongoing clinical trials are using anti-CD40L monoclonal antibodies or specific ligands lacking the Fc segment. A study designed a CD40L-specific peptide ligand, A25, to modify methotrexate-loaded liposomes for treating EAE, which significantly reduced the proportion of CD40L⁺ T cells in EAE mice and effectively inhibited disease progression. Therefore, using A25 as a CD40L-specific peptide ligand can effectively target CD40L⁺ T cells, thereby enhancing drug efficacy.

The A25-Lamp2b lentivirus and empty lentivirus (as shown in Panel A of FIG. 4) were both purchased from OBiO Technology. MSCs were infected with different MOI transfection multiplicity values (3, 15, 30). After 48 hours, green fluorescent protein (GFP) expression could be detected by fluorescence microscopy (Panel B of FIG 4). qPCR results showed that, compared to the group without virus transfection and empty virus transfection group, the expression of the target gene Lamp2b was increased in the MSCs of A25-Lamp2b virus transfection group, with statistically significant differences (Panel C of FIG. 4). Additionally, Western blot results revealed that the expression of Lamp2b and Flag-tagged protein in MSCs of the A25-Lamp2b overexpression virus transfection group was significantly superior to that in MSCs of the empty virus transfection group, with statistically significant differences (Panel D of FIG. 4).

### 3.2 Preparation and Identification of sIVs_{A25}@IL-10

Transmission electron microscopy results showed that sIVs and sEVs obtained by ultracentrifugation exhibited relatively uniform sizes, appearing as spherical or cup-shaped structures with diameters ranging from 70-150 nm (Panel A of FIG. 5). The diameter of the extracted sEVs was analyzed by Nanosight technology. Statistical data indicated that the average diameters of sEVs-GFP, sEVs-A25, sIVs-GFP, and sIVs-A25 were approximately 154.8 ± 1.3 nm, 154.0 ± 1.3 nm, 128.7 ± 0.8 nm, and 119.1 ± 2.2 nm, respectively; the D50 values were 146.9 nm, 146.1 nm, 113.2 nm, and 100.7 nm, respectively; and the D90 values were 215.4 nm, 209.4 nm, 194.2 nm, and 175.2 nm, respectively (Panel B and Panel C of FIG. 5). ELISA results showed that the concentration of IL-10 protein was higher in the sIVs group compared to the sEVs group (Panel D of FIG. 5), indicating that the drug loading capacity of sIVs was superior to that of sEVs. WB results showed that the expression levels of Lamp2b in sIVs were higher than those in sEVs under both normal and overexpression conditions. Under overexpression condition, the expression level of Flag-tagged protein in sIVs-A25 was higher than that in sEVs-A25 (Panel E and Panel F of FIG. 5).

### 4. Summary

MSCs were successfully transfected using lentivirus overexpressing A25-Lamp2b to obtain the targeted modified sIVs and sEVs. The expression level of Lamp2b in sIVs-N was higher than that in sEVs-N, indicating that sIVs were more suitable for targeted modification. The expression levels of Lamp2b and Flag-tagged protein in sIVs-A25 were both higher than those in sEVs-A25. Furthermore, the drug loading efficacy of sIVs was superior to that of sEVs, indicating that sIVs had better performance as drug carriers than sEVs.

### Example 4: Changes in CD40L Expression in an In Vitro T Cell Model and an Experimental Autoimmune Uveitis Animal Model

### 1. Experimental Animals

Seven-week-old female C57BL/6J mice provided by GemPharmatech Co., Ltd., China were used to construct the EAU animal model. All experimental mice were screened to exclude ocular and systemic pathologies before use. The mice were housed in the animal facility (SPF grade) of the Research Institute of Tianjin Medical University Eye Hospital, with environmental conditions set as: stable temperature of (23 ± 2) °C, humidity of (55% ± 10%), and a 12-hour light/dark cycle. Mice in each group were numbered in advance and grouped according to a generated random number table. During the experiment, procedures were strictly conducted in accordance with the standard protocols of the Animal Care and Use Committee of Tianjin Medical University.

### 2. Experimental Methods

### 2.1. Differences in CD40L Expression in an In Vitro T Cell Model

### 2.1.1. Isolation of Mouse Naïve CD4⁺ T Cells

Spleen and draining lymph nodes obtained from a mouse were placed on a copper grid and ground with a syringe plunger. Red blood cells were lysed; then single cells were obtained by centrifugation, resuspended in a 15 mL centrifuge tube: 90 µL of cold MACS buffer per 10⁷ cells, and 10 µL of anti-CD4 magnetic beads per 10⁷ cells, and incubated in the 15 mL centrifuge tube for 15-30 min. The cold MACS buffer was added to the 15 mL centrifuge tube to a volume of 15 mL and centrifuged; then cells were resuspended in 5 mL of cold MACS buffer. The cells were slowly added to a magnetic column pre-rinsed with a MACS buffer placed on a magnetic stand, and rinsed for three times with 5 mL of MACS buffer. The magnetic column was removed from the magnetic stand, placed over a 15 mL centrifuge tube, and added with 5 mL of MACS buffer; then all the liquid in the magnetic column was rapidly pushed into the centrifuge tube using a plunger to obtain naïve CD4⁺ T cells, which were used for *in vitro* activation, proliferation, and differentiation assays.

### 2.1.2. In Vitro T Cell Activation Stimulation Model

During the pathogenesis of autoimmune uveitis, naïve T cells encounter retinal antigens *in vivo* and are activated into antigen-specific T cells (Th1 and/or Th17 cells), which undergo differentiation and proliferation, enter the eye via draining lymph nodes and/or the spleen, recognize intraocular antigens, being reactivated, and further recruit T cells, monocytes/macrophages, neutrophils, and other cells to accumulate within the eye through secreted adhesion molecules, chemokines, etc., thereby amplifying the inflammatory response and leading to damage of intraocular parenchymal cells. The upregulation of CD40L expression in activated T cells serves as the basis for the targeting design in this example.

Both anti-CD3 & CD28 and PMA & Ionomycin are commonly used stimulating factors for *in vitro* T cell activation. Studies have shown that inducing T cell activation and proliferation requires two signals: one is the specific antigen stimulation signal generated by the binding of TCR/CD3 to the specific MHC II antigen peptide complex on the surface of antigen-presenting cells (APCs); the other is a non-specific co-stimulatory signal generated by the interaction between co-stimulatory molecules on the surface of APCs and the corresponding receptors on T cells. Among these, CD28/B7 is the most important co-stimulatory molecule, which can increase IL-2 production, accelerate T cell proliferation, and prevent cells from entering an unresponsive state or apoptosis. A combination use of CD3 and CD28 antibodies *in vitro* to stimulate T cells simulates the dual signal effect of T cell activation, which is currently the most widely used method for T cell activation and expansion. Additionally, within the cell, activation of Protein kinase C (PKC) can induce phosphorylation of numerous downstream protein kinases, forming a cascade reaction that leads to the expression of various proteins and subsequently triggers cell activation. PKC can be activated by the combined action of diacylglycerol ( DAG) and Calcium (Ca²⁺). The phorbol 12-myristate 13-acetate (PMA) is a DAG analog, while Ionomycin is a Ca²⁺ transporter that can transport Ca²⁺ from organelles to the cytoplasm. Therefore, the combined action of PMA and Ionomycin can activate PKC, thereby triggering a series of downstream reactions.

Consequently, 6 ng/mL PMA and 1 µg/mL ION, or 10 µg/mL anti-CD3 and 5 µg/mL anti-CD28 were added to the above culture system of the sorted naïve CD4⁺ T cells for stimulation for 6-12 h. Cells were respectively collected, washed twice with PBS, stained with the corresponding anti-CD40L antibodies, and then analyzed by flow cytometry to detect changes in the proportion of CD40L⁺ T cells.

### 2.2. Changes in CD40L Expression in Experimental Autoimmune Uveitis Animal Model

### 2.2.1. Induction of Experimental Autoimmune Uveitis (EAU) Animal Model

EAU is an animal model of T cell-mediated autoimmune disease, and CD4⁺ T cells are the primary immune cell type showing upregulated CD40L expression following EAU onset. Female C57BL/6 mice aged 6-8 weeks were selected. Antigen polypeptide IRBP₆₅₁₋₆₇₀ and Complete Freund's Adjuvant were added at a 1:1 ratio to two syringes connected with a three-way stopcock. The final volume per mouse was 200 µL, with an antigen dose of 300 µg per mouse. After thorough mixing on ice, 200 µL of the emulsion was subcutaneously injected at six sites on the lateral abdomen and tail base of each mouse. Thirty minutes prior to the subcutaneous injection of the emulsion, PTX was administered intraperitoneally at a dose of 1 µg per mouse.

### 2.2.2. Detection of CD40L Expression Changes

Flow Cytometry: Spleens and lymph node tissues were collected from the EAU mice on day 13 and the agematched naïve mice to prepare single-cell suspensions. The single-cell suspensions were stained with the corresponding anti-CD40L antibodies, and then detected by flow cytometry for changes in the proportion of CD40L⁺ T cells.

Western Blot: Proteins were extracted from the spleens and lymph node tissues of EAU mice on day 13 and agematched naïve mice, loaded with protein loading buffer, denatured at 95°C in a metal bath, and then detected by Western Blot. The Western Blot experimental procedure was the same as previously described.

### 2.3 Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results

### 3.1 CD40L Expression was Upregulated in the In Vitro T Cell Activation Stimulation Model

As shown in Panel A, Panel B, and Panel C of FIG. 6, after *in vitro* stimulation, the proportion of CD40L⁺ cells among CD4⁺ T cells was significantly increased compared to the unstimulated group.

### 3.2 CD40L Expression was Upregulated in the Spleens and Lymph Nodes of EAU Mice

Flow cytometry results in Panel D and Panel E of FIG. 6 showed that, after EAU induction, the proportion of CD40L⁺ cells among CD4⁺ T cells in the spleens and draining lymph nodes of mice was significantly increased compared to the non-modeled group; Western Blot results in Panel F and Panel G of FIG. 6 showed that CD40L protein expression in the spleens of mice after EAU induction was upregulated compared to the non-modeled group.

### 4. Summary

The results indicated that CD40L expression was significantly upregulated after T cell activation. Therefore, targeting the CD40/CD40L pathway holds promise as a novel therapeutic strategy for autoimmune diseases.

### Example 5: Functional Evaluation of sIVs-A25 and sEVs-A25 Targeting T Cells

### 1. Experimental Instruments and Materials

### 1.1 Experimental Reagents

CFSE (Invitrogen, USA); RPMI 1640 Culture Medium (Gibco, USA); Glutamine (Gibco, USA); Fetal Bovine Serum ( FBS) (Gibco, USA); Magnetic bead positive selection mouse Naive CD4 Kit (ebioscience, USA); Magnetic bead positive selection mouse Total CD4 Kit (ebioscience, USA); IL-10 Protein (MCE, USA); BSA (Beyotime, China); Phosphate Buffered Saline (PBS) (Gibco, USA); DiD (Beyotime, China); DAPI (Sigma, USA); Anti-fade Mounting Medium (Solarbio, China); Adhesion Slides (Citotest, China); Polybrene (Gibco, USA); CD3 Antibody (Thermo Fisher Scientific, USA); CD28 Antibody (Thermo Fisher Scientific, USA); 15 mL/50 mL Centrifuge Tubes (Corning, USA); 48-well Plate, 96-well Plate (Corning, USA); LS Column (Miltenyi, Germany); 1 mL, 50 mL Syringes (Ganfa, China); 1.5 mL EP Tube (Axygen, USA); Cell Counting Chamber (Thermo Fisher Scientific, USA); 0.45 µm, 0.22 µm Filters (Sigma, USA).

### 1.2 Experimental Instruments

Water Bath (Tianjin Honour Instrument Co., Ltd, China); Pipettes (Eppendorf, Germany); Benchtop Centrifuge (Eppendorf, Germany); Confocal Microscope (Ceiss, USA); Inverted Phase Contrast Microscope (OLYMPUS, Japan); High-pressure Steam Sterilizer (SAKURA, Japan); Electronic Balance (Sartorius, Germany); Flow Cytometer (BD, USA).

### 2. Experimental Methods

### 2.1 In Vitro Targeting Verification of sIVs-A25

### 2.1.1 CFSE Labeling of sIVs and sEVs

The sIVs and sEVs suspensions were diluted in PBS to 400 µL. CFSE was added to the diluted suspension to a final concentration of 5 µM, co-incubated at room temperature for 10 min, neutralized for excess dye with a large volume of PBS, centrifuged at 11000 g for 70 min to wash once, resuspended in an appropriate amount of PBS, and filtered through a 0.22 µm filter. The sIVs and sEVs were the sIVs and sEVs obtained in section 2.2.1of Example 3.

### 2.1.2 DiD Labeling of sIVs and sEVs

The sIVs resuspension was diluted in PBS to 400 µL. A resulting sIVs dilution was added with DID to achieve a final DID concentration of 5 µg/mL, incubated at 37°C in the dark for 30 min or more. A resulting DiD suspension was added to a 100 kDa ultrafiltration tube, and centrifuged at 14000 g for 15 min. The ultrafiltration tube was added with 400 µL PBS to resuspend pellet, centrifuged at 14000 g for 15 min, added with 50 µL PBS to resuspend the pellet, inverted, replaced with a new collection tube, and centrifuged at 1000 g for 2 min. A resulting mixture was transferred to a new 1.5 mL EP tube and centrifuged at 14000 g for 3 min to collect a supernatant.

### 2.1.3 Co-culture with T Cells

Naïve CD4⁺ T cells were isolated using a magnetic bead negative selection kit, and seeded in a 96-well plate pre-coated with CD3 antibodies at 2x10⁵ cells/well. The cells were incubated in a cell incubator for 24h, and then added with each group of nanovesicles at 20 µg/mL for continued culture. The cells were respectively incubated for 1h, 6h, 12h, and 24h, and then collected for flow cytometry and confocal microscopy analysis.

### 3. Experimental Results

### 3.1 sIVs were More Readily Internalized by T Cells Compared to sEVs

As shown in FIG. 7, after co-incubation of sEVs-GFP, sEVs-A25, sIVs-GFP, sIVs-A25 with CD4⁺ T cells, flow cytometry and confocal microscopy results showed that the proportion of T cell uptake of A25-modified sEVs and sIVs was higher than that of their corresponding unmodified groups, and the T cell uptake proportion of sIVs-A25 was significantly higher than that of sEVs-A25.

### 4. Summary

The above experimental results indicated that sIVs were more readily internalized by T cells compared to sEVs. Moreover, A25 modification significantly increased the quantity of sIVs and sEVs taken up by T cells. Compared to sEVs, the modification of sIVs more significantly improved T cell targeting ability.

### Example 6: Inhibitory Effect of sIVs_{A25}@IL-10 on CD4⁺ In Vitro T Cell Proliferation and Its Promoting Effect on In Vitro Treg Cell Differentiation

### 1. Experimental Instruments and Materials

### 1.1 Experimental Reagents

CFSE (Invitrogen, USA); RPMI 1640 Culture Medium (Gibco, USA); Glutamine (Gibco, USA); Fetal Bovine Serum ( FBS) (Gibco, USA); Magnetic bead positive selection mouse Naive CD4 Kit (ebioscience, USA); Magnetic bead positive selection mouse Total CD4 Kit (ebioscience, USA); IL-10 Protein (MCE, USA); BSA (Beyotime, China); Phosphate Buffered Saline (PBS) (Gibco, USA); CD3 Antibody (Thermo Fisher Scientific, USA); CD28 Antibody (Thermo Fisher Scientific, USA); 15 mL/50 mL Centrifuge Tubes (Corning, USA); 48-well Plate, 96-well Plate (Corning, USA); LS Column (Miltenyi, Germany); 1 mL, 50 mL Syringes (Ganfa, China); 1.5 mL EP Tube (Axygen, USA); Cell Counting Chamber (Thermo Fisher Scientific, USA); 0.45 µm, 0.22 µm Filters (Sigma, USA).

### 1.2 Experimental Instruments

Water Bath (Tianjin Honour Instrument Co., Ltd, China); Benchtop Centrifuge (Eppendorf, Germany); Inverted Phase Contrast Microscope (OLYMPUS, Japan); High-pressure Steam Sterilizer (SAKURA, Japan); Electronic Balance (Sartorius, Germany); Flow Cytometer (BD, USA).

### 2. Experimental Methods

### 2.1 In Vitro T Cell Proliferation Assay (CFSE Method)

96-well plate pre-treatment (plating): CD3 antibodies were diluted in PBS to a concentration of 10 µg/mL, and added to a 96-well plate with 100 µL per well. The perimeter wells of the 96-well plate were added with 100 µL PBS to prevent excessive evaporation. The 96-well plate was incubated overnight at 4°C or incubated in a cell incubator at 37°C for 2 hours. The antibodies were aspirated, and the wells were washed twice with PBS for future use.

Magnetic bead selection of naïve CD4⁺ T cells from mouse spleen. Naïve CD4⁺ T cells were adjusted to 5 mL in a centrifuge tube, added with 1 µL CFSE stock solution (5 mmol/L), mixed rapidly, incubated at room temperature in the dark for 10 min, added with an equal volume of RPMI1640 culture medium containing 10% FBS to stop staining, centrifuged, and then resuspended in complete medium (composition: 90% 1640 basal culture medium + 10% FBS + 2 µg/mL CD28 antibody + 100 U/mL penicillin-streptomycin + 50 µM β-mercaptoethanol). Cells were seeded at 3x10⁵ cells/well in the pretreated 96-well plate. A blank control group and various nanovesicle treatment groups were set up, with three replicate wells per group. After 72 hours of culture, cells were collected and added with CD4 antibody for staining. CFSE fluorescence expression was detected by flow cytometry.

### 2.2 In Vitro Treg Differentiation Assay

### 2.2.1 In Vitro Treg Differentiation

Isolation of naïve CD4⁺ T cells was performed according to the instructions of the magnetic bead negative selection kit. After the isolation, 2x10⁵ cells/well were seeded in a 96-well plate pre-coated with CD3 antibody and cultured in a cell incubator for 5 days according to the following differentiation conditions: X-VIVO 15 medium + 2 µg/mL CD28 antibody + 5 ng/mL TGF-β + 20 ng/mL IL-2. A blank control group and various nanovesicle treatment groups were set up, with three replicate wells per group. Flow cytometry detection was performed after 5 days.

### 2.2.2 Flow Cytometry Detection of Treg Cell Proportion

Cells to be tested were placed into flow cytometry tubes, added with PBS, centrifuged at 400 g for 5 min, resuspended in 100 µL PBS, added with 1 µL of CD4-BV711 antibody for staining, incubated in the dark for 30 min, added with PBS, centrifuged at 400 g for 5 min, sequentially added with fixation/permeabilization buffer for fixation and permeabilization treatment, added with 1 µL of FOXP3-PE antibody for staining, incubated in the dark for 30 min, centrifuged at 400 g for 5 min, and resuspended in 500 µL PBS. The proportion of FOXP3⁺CD4⁺ cells was detected by flow cytometry.

The sEVs_{A25}@IL-10, sIVs_{GFP}@IL-10, and sIVs_{A25}@IL-10 used in the experiments were obtained as described in Example 3.

### 2.3 Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results

### 3.1 In Vitro T Cell Proliferation Assay

When sEVs_{A25}@IL-10, sIVs_{GFP}@IL-10, and sIVs_{A25}@IL-10 were used at a concentration of 10 µg/mL, they were able to inhibit T cell proliferation stimulated by CD3/CD28 antibodies to a certain extent (Panel A and Panel C of FIG. 8). Wherein, the inhibitory effect of the sIVs_{GFP}@IL-10 group on *in vitro* T cell proliferation was significantly superior to that of the sIVs_{GFP}@IL-10 group and the sEVs_{A25}@IL-10 group, with statistically significant differences. This indicated that targeted modification of sIVs enhanced the targeted delivery efficiency of the loaded drug IL-10, thereby improving the inhibitory effect of IL-10 on T cell proliferation stimulated by CD3/CD28 antibodies.

### 3.2 Treg Cell Differentiation Assay

Treg cells are the primary inhibitory cell population *in vivo,* playing a crucial role in graft tolerance, suppression of autoimmune responses, and maintenance of immune homeostasis. In autoimmune diseases, Treg cells can suppress pathogenic T cells and promote disease regression. Their dysfunction and dysregulation can lead to autoimmune diseases.

The results in Panel B and Panel D of FIG. 8 showed that the sIVs_{GFP}@IL-10 group had no significant promoting effect on Treg differentiation. However, the sIVs_{A25}@IL-10 group and the sEVs_{A25}@IL-10 group exhibited a significant promoting effect on the process of T cells differentiation into Treg cells, and this difference was statistically significant (P < 0.05). This indicated that targeted modification of sIVs enhanced the targeted delivery efficiency of the loaded drug IL-10, thereby improving the promoting effect of IL-10 on TGF-β-induced Treg cell differentiation.

### 4. Summary

By encapsulating IL-10 in sIVs and sEVs, the aim was to enhance the inhibitory capacity of MSC-derived sIVs and sEVs on *in vitro* T cell proliferation and promote *in vitro* Treg cell development, thereby achieving a more significant immunosuppressive effect. The above experimental results showed that sIVs_{A25}@IL-10 could not only effectively inhibit the *in vitro* proliferation triggered by CD3/CD28 antibody stimulation but also promote the differentiation of naive CD4⁺ T cells into Treg cells. Furthermore, the effect of targeted modified sIVs was more pronounced than that of unmodified sIVs, and the targeted modified sIVs exhibited comparable immunosuppressive efficacy to sEVs.

### Example 7: Therapeutic Effect of sIVs_{A25}@IL-10 on Experimental Autoimmune Uveitis

### 1. Experimental Subjects

### 1.1 Experimental Animals

Seven-week-old female C57BL/6 mice provided by GemPharmatech Co., Ltd. , China were used to construct the EAU animal model. All experimental mice were screened to exclude ocular and systemic pathologies before use. The mice were housed in the animal facility (SPF grade) of the Research Institute of Tianjin Medical University Eye Hospital, with environmental conditions set as: stable temperature of (23 ± 2) °C, humidity of (55% ± 10%), and a 12-hour light/dark cycle. Mice in each group were numbered in advance and grouped according to a generated random number table. During the experiment, procedures were strictly conducted in accordance with the standard protocols of the Animal Care and Use Committee of Tianjin Medical University.

### 2. Experimental Methods

### 2.1 EAU Induction

Female C57BL/6 mice aged 6-8 weeks were selected. Antigen polypeptide IRBP₆₅₁₋₆₇₀ and Complete Freund's Adjuvant were added at a 1:1 ratio to two syringes connected with a three-way stopcock. The final volume per mouse was 200 µL, with an antigen dose of 300 µg per mouse. After thorough mixing on ice, 200 µL of the emulsion was subcutaneously injected at six sites on the lateral abdomen and tail base of each mouse. Thirty minutes prior to the subcutaneous injection of the emulsion, PTX was administered intraperitoneally at a dose of 1 µg per mouse.

### 2.2 In Vivo Efficacy Verification of sIVs_{A25}@IL-10

### 2.2.1 Treatment and Grouping of EAU Model Mice

On day 11 after modeling, EAU model mice were randomly divided into groups, ensuring each group contained 6 mice. Mice in each group received tail vein injections of 1 µg IL-10, 30 µg sIVs_{A25}@IL-10, 30 µg sIVs_{GFP}@IL-10, and 30 µg sEVs_{A25}@IL-10, respectively. Similarly, in the blank control group, mice were injected with an equal volume of phosphate-buffered saline (PBS) to ensure consistency of experimental conditions. The sEVs_{A25}@IL-10, sIVs_{GFP}@IL-10, and sIVs_{A25}@IL-10 used in the experiments were obtained as described in Example 3.

### 2.2.2 Clinical/Pathological Inflammation Scoring of Experimental Autoimmune Uveitis

Starting from day 9 after model induction, the severity of disease in each group of mice was observed every 2 days using an indirect ophthalmoscope. Mouse eyeballs were enucleated at different stages of the disease, fixed, and prepared for paraffin sections. Routine HE staining was performed. The extent of retinal tissue structural damage in mice was recorded under an optical microscope. Clinical fundus and histopathological scores were recorded according to the Caspi grading standard.

### 2.2.3 Mouse OCT and Fundus Imaging Examination

Starting from day 10 after model induction, changes in the fundus structure of mice were observed every 4 days using a Heidelberg SPECTRALIS-OCT (Germany). The pupils of both eyes of the mice were dilated using tropicamide and epinephrine eye drops. Then, the mice were anesthetized via intraperitoneal injection of avertin. After the mice reached a stable anesthetic state, sodium hyaluronate gel was administrated to the eyes of mice, and retinal scan was performed. Scanning images were centered on the mouse optic disc, with one image captured for each of the horizontal and vertical sections.

### 2.2.4 Electroretinography (ERG)

On day 25 after modeling, EAU mice from each group were subjected to dark adaptation for more than 12 hours in a darkroom. The electrophysiological response of the retinas from mice in each group under dark adaptation was tested. The mice were subjected to dark adaptation for 12 hours, anesthetized intraperitoneally, and their pupils were fully dilated. Following anesthesia of the ocular surface, the mice were placed on a 37°C constant-temperature operating table. A red electrode needle was inserted into the skin of the neck of mouse, with the needle tip pointing towards the head. A green electrode needle was inserted into the tail of mouse. The electrodes were fixed with tape. Gatifloxacin eye gel was administrated to the cornea to maintain moisture of the cornea and prevent infection from instrument-induced corneal abrasion. The angle was adjusted to align the cornea with the corneal electrode. According to the standardized protocol of International Society for Clinical Electrophysiology of Vision (ISCEV), stimuli were given to the mice from low to high intensities, and the electroretinogram was recorded. The stimulus intensity range was from -1.7 to 3.1 log (cd•s/m²). Stimuli were given according to low (-1.7~0.4), medium (0.7~2.2), and high stimulus intensities (2.5~3.1), with 7, 5, and 3 repetitions respectively, and the average of single flash was recorded. The interstimulus intervals were 15, 30, and 60 seconds, respectively.

### 2.2.5 Isolation of Cells from Draining Lymph Nodes and Spleen Tissue and Determination of T Cell Subsets by Flow Cytometry

On day 17 after EAU mouse model induction, draining lymph nodes and spleen tissues of EAU mice from each group were collected, ground, and isolated to obtain single-cell suspensions. The single-cell suspension was divided into two parts.

One part was subjected to intracellular staining. Under co-stimulation with PMA, ION, and BFA, the cells were cultured in a CO₂ incubator for 6h, collected, thoroughly washed with PBS, added with Live/Dead antibody and CD4 antibody for staining, incubated at 4°C in the dark for 30 min, washed, added with fixation buffer and fixed at room temperature for 30 min, centrifuged, added with a permeabilization buffer, and washed twice. Then, a resulting sample was respectively added with the permeabilization buffer and IFN-γ, IL-4, IL-17A antibodies for intracellular staining, and incubated at 4°C in the dark for 30 min. After staining, the permeabilization buffer was thoroughly washed, the cells were resuspended in PBS for analysis on the BD FacsCelesta to detect changes in Th1, Th2, and Th17 cell subsets.

The other part of unstimulated cells was subjected to FOXP3⁺CD25⁺ CD4⁺ T cell nuclear staining. The cell sample was respectively added with Live/Dead antibody and CD4⁺ antibody for staining, incubated at 4°C in the dark for 30 min, washed, added with a fixation buffer for nuclear membrane permeabilization and fixed at room temperature for 30 min, centrifuged, added with a nuclear membrane permeabilization buffer, and washed twice. Then, a resulting sample was respectively added with the membrane permeabilization buffer and FOXP3⁺CD25⁺ antibodies for intranuclear staining, and incubated at 4°C in the dark for 30 min. After staining, the membrane permeabilization buffer was thoroughly washed, the cells were resuspended in PBS for analysis on the BD FacsCelesta to detect changes in Treg cell subsets.

### 2.3 Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results

### 3.1 sIVs_{A25}@IL-10, Similar to sEVs_{A25}@IL-10, Significantly Alleviated Fundus Inflammation in EAU Mice

Results showed that the fundus inflammation scores and pathological scores in the three treatment groups were lower compared to the control group (P < 0.05) (FIG. 9). Among them, the therapeutic effects of the sEVs_{A25}@IL-10 and sIVs_{A25}@IL-10 treatment groups were comparable, with significant improvement in fundus inflammation, reduced optic disc edema, disappearance of vascular white sheathing, and decreased vitreous inflammatory cells. HE sections showed that the retinal structure was restored to a flattened state, and the structure of all layers of the retina was superior to that of the sIVs_{GFP}@IL-10 treatment group, with statistically significant differences. OCT results were consistent with this finding.

### 3.2 sIVs_{A25}@IL-10 Exhibited Stronger Neuroprotective Effect on EAU Mice

As shown in FIG. 10, the unmodified sIVs_{GFP}@IL-10 group could significantly increase the a-wave and b-wave amplitudes under high stimulation compared to the PBS and IL-10 groups. The modified sIVs could significantly increase the a-wave and b-wave amplitudes compared to the modified sEVs. This indicated that the modified sIVs had superior neuroprotective effects.

### 3.3 sIVs_{A25}@IL-10, Similar to sEVs_{A25}@IL-10, could Increase the Proportion of Treg Cells in Draining Lymph Nodes and Reduce the Proportion of Th1 Cells in Both Draining Lymph Nodes and Spleen

As shown in FIG. 11, the proportion of Treg (CD4⁺CD25⁺FOXP3⁺) cells in the draining lymph nodes of mice in the modified sIVs_{A25}@IL-10 and sEVs_{A25}@IL-10 treatment groups was significantly increased compared to the PBS, IL-10, and sIVs_{GFP}@IL-10 groups; and the proportion of Th1 (CD4⁺IFN-γ⁺) cells in the draining lymph nodes and spleens of mice in the modified sIVs_{A25}@IL-10 and sEVs_{A25}@IL-10 treatment groups was significantly reduced compared to the PBS, IL-10, and sIVs_{GFP}@IL-10 groups, indicating that the modified sIVs improved the tissuetargeted delivery efficiency of IL-10, enhanced the promoting effect of IL-10 on Treg cell differentiation in the draining lymph nodes of EAU mice, and strengthened the inhibitory effect of IL-10 on Th1 cell proliferation in the draining lymph nodes and spleens of EAU mice as well.

### 4. Summary

After EAU mice were administered sIVs_{A25}@IL-10 via tail vein injection, the severity of the disease was significantly improved. HE staining sections also showed recovery of retinal structure and reduced infiltration of inflammatory cells. This demonstrated that tail vein injection of sIVs_{A25}@IL-10 could effectively inhibit the progression of inflammation in EAU mice. Observation of ERG electrophysiological results indicated that sIVs_{A25}@IL-10 possessed significant retinal neuroprotective functions. Flow cytometry results suggested that sIVs_{A25}@IL-10 may inhibit the progression of inflammation in EAU mice by promoting Treg cell differentiation in the draining lymph nodes and spleens.

### Example 8: Lamp2b Expression in Cellular sIVs was Higher than in sEVs

### 1. Experimental Methods

The specific experimental process was shown in FIG. 12.

### 1.1 Cell Culture

The extraction and culture of cells were the same as in Example 1.

### 1.2 Extraction of sIVs

When the cells reached 90% confluence, the supernatant was completely aspirated. PBS was added to wash the cells twice. Trypsin was then added to digest the cells. After digestion, PBS was used to neutralize and wash the cells three times. Cell counting was subsequently performed, and cell concentration was adjusted to 1×10⁶ cells/mL using PBS. 2 mL of a resulting cell suspension with a density of 1×10⁶ cells/mL was taken and added to the bottom of a 50 mL centrifuge tube. An ultrasonic probe was placed at the center of liquid surface for ultrasonic treatment. During the ultrasonic treatment, an ultrasonic amplitude parameter was set to 20%, a time parameter was set to 15 s, with a cycle of 2 s on and 2 s off, and the centrifuge tube was placed on ice. Subsequently, a resulting liquid was transferred to a 2 mL centrifuge tube for centrifugation.

A resulting supernatant was filtered using a 50 mL syringe and a 0.45 µm filter (the filter membrane was pre-wet with PBS). IVs were extracted and isolated using differential centrifugation at an RCF of 150,000 for 70 min at a temperature of 4°C. A resulting pellet from each tube was resuspended in 60 µL of clean PBS, and collected in an EP tube. Protein quantification was performed using the BCA assay. Then, protein denaturation was performed for Western Blot experiments.

### 1.3 Collection of sEVs

FBS is an essential component for cell culture *in vitro*; however, it contains a large number of bovine-derived extracellular vesicles, which are also present in complete cell culture medium containing FBS. To remove bovine extracellular vesicles, FBS was centrifuged at 4°C at 110,000 × g overnight (approximately 12 hours). When cells reached 60% confluence, the cells were cultured in complete medium containing 10% exosome-depleted FBS for 48 hours. Then, supernatant was collected, and extracellular vesicles were isolated by ultracentrifugation at 4 °C. The specific steps comprised ultracentrifugation at 300 g × 10 min, 2000 g × 10 min, 10000 g × 30 min, and two rounds of 110000 g × 70 min. A resulting pellet was resuspended in PBS to yield sEVs predominantly composed of exosomes, and collected in an EP tube. Protein quantification was performed using the BCA assay. Then, protein denaturation was performed for Western Blot experiments.

The collection of small intracellular vesicles from 293T cells and HeLa cells, as well as the Western Blot experimental procedure were the same as above.

### 2. Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results

### 3.1 Lamp2b Expression in sIVs from MSCs, 293 Cells, and HeLa Cells was Higher than in sEVs

In this experiment, Western Blot experiment was performed on cellular proteins, sIVs proteins, and sEVs proteins from three cell types (MSCs, 293 cells, and HeLa cells) to detect Lamp2b protein expression (FIG. 13). The results showed that the protein expression of Lamp2b in sIVs was significantly higher than that in sEVs.

### 4. Summary

This example demonstrated that the expression of Lamp2b in sIVs from MSCs, 293T cells, and HeLa cells was higher than in sEVs, indicating that the Lamp2b expression in cellular sIVs was higher than that in sEVs.

### Example 9: Preparation of sIVs_{RGD}@PEDF

### 1. Experimental Methods

### 1.1 Isolation, Culture, and Lentiviral Infection of MSCs

MSCs were extracted from human umbilical cord via digestion and centrifugation, and obtained and purified by differential attachment method. MSCs were transfected with lentivirus to construct MSC-RGD. P3 generation UMSCs in good condition were taken, and subjected to lentiviral transfection after 12h when cell confluence reached 50%-70%. Lentiviral transfection solution was prepared as follows: six 50 mL centrifuge tubes were each added with half the volume of complete medium of the normal culture system, added with polybrene transfection reagent to a final concentration of 6 µg/mL. For the experimental groups, corresponding volumes of Lamp2b-RGD lentivirus calculated based on MOI values of 10, 30, and 50 were respectively added. For the control groups, control lentivirus at the same MOI gradients were respectively added. A resulting liquid was pipetted to mix thoroughly. The original cell culture medium was aspirated. The above viral transfection solutions were respectively added, gently shaken to ensure uniform liquid coverage on the cell surface. The cells were gently placed in a cell incubator for incubation, added with complete medium 6-hour later to reach the full volume of normal culture system, added with the lentiviral transfection solution to transfect for 24 hours, and then changed with new medium. The infection procedure for 293T cells was the same as above.

### 1.2 Collection and Storage of sIVs

When the UMSCs reached 90% confluence, the medium was aspirated at first to remove the supernatant; then the cells were washed with warm PBS, added with 3 mL trypsin to digest for 3 min, neutralized with 6 mL PBS, centrifuged at 1000 g for 5 min, resuspended in PBS and centrifuged for washing for twice, resuspended in PBS, and counted using an automated cell counter. A resulting cell suspension was diluted with PBS to a density of 1*10⁶/mL. Subsequently, IVs were obtained by disrupting cells with an ultrasonic machine. Ultrasonic treatment was performed on ice on 2 mL of aliquot each time (2 mL, power: 20%-30%, 2s/2s, 15s-120s). A resulting liquid after the ultrasonic treatment was collected in a 2 mL EP tube and centrifuged at 2000 g for 10 min followed by 20,000 g for 30 min. A resulting supernatant was collected, transferred to a new 15 mL centrifuge tube, labeled with the date, and stored at - 80°C.

The collection procedure of small intracellular vesicles from 293T cells was the same as above.

### 1.3 Extraction of sIVs

The supernatant was filtered using a 50 mL syringe and a 0.45 µm filter (the filter membrane was pre-wet with PBS). IVs were extracted and isolated using differential centrifugation at an RCF of 150,000 for 70 min at a temperature of 4°C. A resulting pellet from each tube was resuspended in 60 µL of clean PBS, and collected in an EP tube. Protein quantification was performed using the BCA assay.

sEVs were obtained according to sections 1.1-1.3 of Example 8 and similar methods. sIVs/sEVs overexpressing Lamp2b-RGD were the targeted modified sIVs/sEVs, and sIVs/sEVs transfected with empty vector virus were the unmodified sIVs/sEVs, hereafter referred to as sIVs-RGD, sEVs-RGD, sIVs-GFP, and sEVs-GFP.

### 1.4 Preparation of sIVs_{RGD}@PEDF

PEDF drug solution was added to sIVs-RGD, mixed well, and left to stand in a 37°C incubator for 1 h. A resulting liquid was added to a 100 kDa ultrafiltration tube and centrifuged to remove free drug. The ultrafiltration tube was pre-wet with PBS, added with the liquid and centrifuged at 14,000 g for 20 min, added with 300 µL of PBS and centrifuged at 14,000 g for 20 min (the step of which was repeated twice), and then inverted and centrifuged at 4000 g for 2 min to recover and obtain sIVs_{RGD}@PEDF, which was temporarily stored at 4°C. Similar methods were used to obtain sIVs_{GFP}@PEDF and sEVs_{RGD}@PEDF. Among these, sIVs_{RGD}@PEDF and sEVs_{RGD}@PEDF represent the targeted modified sIVs and sEVs loaded with PEDF, while sIVs_{GFP}@PEDF and sEVs_{GFP}@PEDF represent the unmodified sIVs and sEVs loaded with PEDF.

### 1.5 Quantification of Drug Loading in sIVs_{RGD}@PEDF by ELISA

Concentration of the loaded drug in sIVs_{RGD}@PEDF was determined using ELISA. A standard curve was generated using a series of drug concentrations ranging from 0 to 6 ng/mL. 100 µL of standard solution and sample solutions were added to the ELISA plate for detection. A standard curve was plotted with drug concentration as the x-axis and absorbance as the y-axis, and the regression equation was calculated. This equation was used to calculate the drug loading rate and encapsulation efficiency.

### 2. Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results

### 3.1 Lamp2b-RGD Lentivirus Successfully Achieved High Expression in MSCs

RGD is widely recognized for its specific targeting of integrins, which are weakly expressed in quiescent endothelial cells and most normal tissues but are strongly and highly expressed in proliferating endothelial cells, especially integrin αv and integrin β3. Therefore, we chose to overexpress RGD in MSCs to collect sIVs and sEVs with high RGD expression, aiming to target the lesion sites in retinal neovascularization model for therapeutic purpose. Lamp2b is a lysosomal membrane protein and a transmembrane protein on the exosome membrane surface. Moreover, in Example 8, we found that Lamp2b expression in cellular sIVs was higher than in sEVs. Therefore, Lamp2b-RGD lentivirus was constructed to transfect MSCs. Flag is a common lentiviral tag protein. Successful detection of Flag expression can also be used to demonstrate successful lentiviral transfection.

In preliminary experiments, MSCs were transfected with lentivirus at multiplicity of infection (MOI) values of 10, 30, and 50. Proteins from MSCs of the normal control group, empty vector group, and virus-transfected groups were extracted, denatured, and subjected to Western Blot experiment. The results showed that the target lentivirus Lamp2b-RGD was successfully overexpressed in MSCs. Western Blot results showed that when the MOI was 30, the overexpression fold of Lamp2b had reached a maximum (FIG. 14). Furthermore, Flag, as a common lentiviral tag protein, was also successfully highly expressed, indicating that Lamp2b-RGD lentivirus was successfully overexpressed in MSCs. When the MOI was 30, the overexpression fold of the Flag-tagged protein had reached a maximum. Subsequent experiments were performed using MOI=30.

### 3.2 In the MSC Group with High Lamp2b-RGD Expression, Lamp2b Expression in sIVs was Higher than in sEVs

By collecting sIVs and sEVs from the normal MSC group, empty vector MSC group, and MSC group with high Lamp2b-RGD expression, and performing protein denaturation and Western Blot experiment, we found that in MSCs of the normal group and empty vector group, Lamp2b expression in sIVs was higher than in sEVs (FIG. 15). Notably, in the MSC group with high Lamp2b-RGD expression, Lamp2b expression in sIVs was still higher than in sEVs (FIG. 15).

### 3.2 Drug Loading Capacity of sIVs was Superior to That of sEVs

The composite formulation prepared by loading PEDF into sEVs_{RGD} was denoted as sEVs_{RGD}@PEDF, and the composite formulation prepared by loading PEDF into sIVs_{RGD} was denoted as sIVs_{RGD}@PEDF. The PEDF content in sEVs_{RGD}@PEDF and sIVs_{RGD}@PEDF was detected by ELISA. The results showed that the concentration of PEDF encapsulated in sIVs was higher than that in sEVs, demonstrating that the drug loading capacity of sIVs was superior to that of sEVs (FIG. 16).

### 4. Summary

This example demonstrated that Lamp2b-RGD lentivirus successfully achieved high expression in MSCs, and in the MSC group with high Lamp2b-RGD expression, Lamp2b expression in sIVs was still higher than in sEVs. The drug loading capacity of sIVs was superior to that of sEVs.

### Example 10: High Expression of RGD-Binding Integrins in In Vivo and In Vitro Models of Neovascularization

### 1. Experimental Methods

### 1.1 Cell Culture

Human HRECs were purchased from Angioproteomie Inc., USA. Preparation of complete cell culture medium was as follows: 5 mL of fetal bovine serum, 1 mL of growth factor, and 1 mL of penicillin-streptomycin were added to 93 mL of ECM basal medium to obtain complete medium containing 5% serum. Cell growth density was observed using a low-magnification microscope, and a high-magnification microscope was used to observe cells as round, oval, or polygonal flat cells with abundant components in the cytoplasm and very small vacuoles within the cells. The cells were seeded in culture flasks and cultured in a 5% CO₂ incubator at 37°C. The medium was changed every 3 days. When cell confluence reached 80%, the cells were passaged at a split ratio of 1:2. Cells at passages 3 to 5 (P3-P5) were used for experiments.

### 1.2 Experimental Animals

8-week-old healthy male C57BL/6 mice weighing 21-22g, SPF grade, were purchased from SiPeiFu Co., Ltd., China (Animal Production License: SCXK (Jing) 2019-0010). All experimental animals were housed at room temperature with a normal diet and 12h of light and dark periods respectively. The animal housing environment and experimental procedures complied with the regulations of the National Science and Technology Commission regarding the "Regulations on Management of Experimental Animals" and were approved by the Institutional Animal Ethics Committee (Ethics Approval No.: TJYY2023120297). Mice were grouped according to a random number table for intravitreal injection.

### 1.3 VEGF-Induced Proliferative Endothelial Cell Model

HRECs were seeded into 12-well culture plates with half-medium (FBS 2.5%/ECG 0.05%), incubated for 12 h, changed with new half-medium, divided into two groups: a normal group and a VEGF group (10 ng/mL), and incubated at 37°C for 24 h. After removing the culture medium, the cells were washed with PBS. RNA and protein were harvested from the cells. PCR and Western Blot methods were used to detect the expression levels of RGD-targeting integrins αV and β3 in VEGF-induced HRECs.

### 1.3 Oxygen-Induced Retinopathy Model (OIR Mice)

Newborn C57BL/6J mice and nursing mothers were exposed to a hyperoxic environment (75% O₂) from postnatal day 7 (P7) for 5 days and returned to room air at P12. The number of newborn mice assigned to each nursing mother was the same. The mothers were alternated between the oxygen chamber and normoxia every 24 h. Mice were provided with a standard diet and water, and randomly assigned to each treatment group. Retinal tissues were collected from the mice on days 12, 14, and 17 to extract RNA and protein. PCR and Western Blot methods were used to detect the expression levels of RGD-targeting integrins αV and β3 in OIR mice.

### 2. Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results

### 3.1 High Expression of RGD-Binding Integrins in VEGF-Induced Endothelial Cells

Vascular endothelial growth factor (VEGF) is a highly specific growth factor that promotes the growth of vascular endothelial cells, exhibiting functions of promoting vascular permeability, migration, proliferation, and angiogenesis of endothelial cells. The VEGF-induced endothelial cell proliferation model is the most common *in vitro* model of retinal neovascularization. RGD is well known for specifically targeting integrins, which are weakly expressed in quiescent endothelial cells and most normal tissues but are strongly and highly expressed in proliferating endothelial cells, especially integrin αv and integrin β3. Therefore, we first examined the expression of integrin αv and integrin β3 in VEGF-induced endothelial cells.

Proteins from VEGF-induced HRECs were collected for Western Blot experiment. The results showed that RGD-binding integrins αV and β3 were highly expressed in VEGF-induced proliferative endothelial cells (FIG. 17).

### 3.2 High Expression of RGD-Binding Integrins in the Retinas of OIR Mice

The oxygen-induced retinopathy (OIR) mouse model utilizes hyperoxia to create non-perfusion areas in the retina, inducing significant upregulation of pro-angiogenic factors, thereby forming neovascularization. It is commonly used to validate the effects of drugs or therapeutic targets for retinal neovascularization.

RNA was collected from the retinas of mice at P12, P14, and P17 for PCR. The results showed that RGD-binding integrin αV was highly expressed in the retinas of OIR mice at P12, P14, and P17, while integrin β3 was highly expressed in the retinas of OIR mice at P14 and P17 (FIG. 18). Proteins were collected from the retinas of mice at P12, P14, and P17 for Western Blot experiment. The results showed that RGD-binding integrin αV was highly expressed in the retinas of OIR mice at P12, P14, and P17, while integrin β3 was highly expressed in the retinas of OIR mice at P17 (FIG. 19).

### 4. Summary

This example demonstrated that RGD-binding integrin αV and integrin β3 were highly expressed in the VEGF-induced endothelial cells and OIR mice, indicating that sIVs and sEVs with high RGD expression could be used for therapeutic efficacy studies.

### Example 11: sIVs-RGD Exhibited Stronger Targeting Ability to Neovascular Endothelial Cells Compared to sEVs-RGD

### 1. Experimental Methods

### 1.1 Cell Culture

Human HRECs were purchased from Angioproteomie Inc., USA. Preparation of complete cell culture medium was as follows: 5 mL of fetal bovine serum, 1 mL of growth factor, and 1 mL of penicillin-streptomycin were added to 93 mL of ECM basal medium to obtain complete medium containing 5% serum. Cell growth density was observed using a low-magnification microscope, and a high-magnification microscope was used to observe cells as round, oval, or polygonal flat cells with abundant components in the cytoplasm and very small vacuoles within the cells. The cells were seeded in culture flasks and cultured in a 5% CO₂ incubator at 37°C. The medium was changed every 3 days. When cell confluence reached 80%, the cells were passaged at a split ratio of 1:2. Cells at passages 3 to 5 (P3-P5) were used for experiments.

### 1.2 Experimental Animals

8-week-old healthy male C57BL/6 mice weighing 21-22g, SPF grade, were purchased from SiPeiFu Co., Ltd., China (Animal Production License: SCXK (Jing) 2019-0010). All experimental animals were housed at room temperature with a normal diet and 12h of light and dark periods respectively. The animal housing environment and experimental procedures complied with the regulations of the National Science and Technology Commission regarding the "Regulations on Management of Experimental Animals" and were approved by the Institutional Animal Ethics Committee (Ethics Approval No.: TJYY2023120297). Mice were grouped according to a random number table for intravitreal injection.

### 1.3 Co-culture of DiD-Labeled sIVs and sEVs with Cells

Vesicles were co-incubated with a lipophilic tracer DiD solution at 37°C for 30 minutes. Excess DiD was removed using an Amicon^{®} Ultra centrifugation filter. Cells were seeded onto round coverslips placed in 24-well culture plates and incubated for 24 hours. The DiD-labeled vesicles were added to the cell culture medium, grouped as follows: sIVs and sEVs collected from UMSCs of empty vector group and Lamp2b-RGD high expression groups (sEVs-GFP, sEVs-RGD, sIVs-GFP, and sIVs-RGD vesicles obtained as described in section 1.3 of Example 9), and incubated at 37°C for different times: 12 hours or 24 hours. After removing the medium, the cells were washed with PBS, fixed with 4% PFA for 10 minutes at room temperature, then incubated with 0.1% Triton X-100 at room temperature for 5 minutes to permeabilize the cells. Subsequently, the cells were stained with CoraLite^{®} Plus 488-conjugated Phalloidin antibody at room temperature for 30 minutes. DAPI was used to label nuclei. Finally, the cells were imaged using a confocal laser scanning microscope, and the amount of internalized vesicles was analyzed by Image J.

### 1.4 Intravitreal Injection of DiD-Labeled Vesicles into OIR Mice

OIR mice were modeled as previously described. Vesicles were stained with DiD as previously described. DiD-labeled vesicles were intravitreally injected into OIR mice at a mass of 1 µg. Eyeballs were enucleated for observation and evaluation at 8 hours, 24 hours, and 5 days post-injection.

### 1.4 Retinal Sectioning

Frozen retinal sections (8 µm thick) were used for immunofluorescence analysis. Retinal sections were fixed with PFA at room temperature for 15 minutes, then the slides were washed with PBS. Nuclei were stained with DAPI. Finally, the slides were mounted with anti-fade mounting medium and observed using a confocal laser scanning microscope.

### 2. Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results

### 3.1 Cellular Internalization Capacity of sIVs-RGD was Superior to That of sEVs-RGD

To evaluate the cellular uptake capacity of the two types of vesicles, HRECs were co-cultured with the aforementioned four groups of vesicles (sEVs-GFP, sEVs-RGD, sIVs-GFP, and sIVs-RGD) for 12 h (FIG. 20) and 24 h (FIG. 21). Briefly, equal amounts of DiD-labeled vesicles were incubated with cells in a normal state and VEGF-induced HRECs. At different time points, confocal microscopy was used to observe the distribution of DiD to determine cellular uptake of vesicles. The results showed that in HRECs, the cellular internalization rate of sIVs consistently exceeded that of sEVs. Notably, the cellular internalization rate of RGD-targeted modified sIVs-RGD was consistently higher than that of sEVs-RGD.

### 3.2 sIVs-RGD Penetrated the Retina Faster and Exhibited Longer Retention Time Compared to sEVs-RGD

To evaluate the retinal uptake of the four groups of vesicles (sEVs-GFP, sEVs-RGD, sIVs-GFP, and sIVs-RGD), equal amounts of DiD-labeled vesicles were administered via intravitreal injection into the eyes of OIR mice, a model of retinal neovascularization. Eyeball samples were enucleated at different time points, and then prepared for frozen sections. Confocal microscopy was used to observe vesicle uptake within the retina.

After intravitreal injection of DiD-labeled vesicles, the vesicles diffused from the vitreous cavity towards the retina. The results showed that 8 hours after injection, sEVs were distributed within the vitreous cavity and had not yet reached the retina, whereas sIVs had already reached the entire retinal layer and were internalized by cells in the ganglion cell layer and inner nuclear layer. Wherein, the internalization rate of sIVs-RGD exceeded that of sIVs-GFP and sEVs-RGD (Panel A and Panel B of FIG. 22). Twenty-four hours after intravitreal injection, the four types of vesicles showed diffuse distribution throughout the retina, and the internalization rate of sIVs-RGD consistently exceeded that of sIVs-GFP and sEVs-RGD (Panel C and Panel D of FIG. 22). Subsequently, the distribution of the four types of vesicles in the retina was observed to be gradually decreased by day 5, but the distribution trend remained consistent with the previously observed results (Panel E and Panel F of FIG. 22). Notably, sIVs-RGD penetrated the retina faster and exhibited a longer retention time compared to sEVs, and the internalization rate of RGD-modified sIVs consistently exceeded that of unmodified sIVs in the retinas of OIR mice.

### 4. Summary

This example demonstrated that the internalization capacity of *in vitro* cultured cells for sIVs was superior to that for sEVs. Compared to unmodified sIVs and RGD-modified sEVs, RGD-modified sIVs exhibited a higher uptake rate for proliferating endothelial cells. By intravitreal injection in mice, it was observed that the internalization capacity of retina for sIVs was superior to that for sEVs, sIVs-RGD penetrated the retina faster and exhibited a longer retention time compared to sEVs, and the internalization rate of RGD-modified sIVs consistently exceeded that of unmodified sIVs in the retinas of OIR mice. This finding fully demonstrated that sIVs exhibited good tissue compatibility, and that sIVs-RGD exhibited a stronger targeting ability to neovascular endothelial cells.

### Example 12: Therapeutic Effects of sIVs_{RGD}@PEDF on Neovascularization Model In Vitro

### 1. Experimental Methods

### 1.1 Proliferation Assay

HRECs were seeded in 96-well plates at a density of 4500 cells/well. A VEGF solution (10 ng/mL) was added to the culture medium. Different groups were designed as follows: the group only added with the VEGF solution served as the positive control group. Groupings included sIVs-GFP, PEDF, sIVs_{GFP}@PEDF, sIVs_{RGD}@PEDF, sEVs_{RGD}@PEDF, with each vesicle source referring to Example 9.

After 24 h of culture for the experimental groups, the proliferation of cells in each group was tested by the CCK method.

### 1.2 Transwell Migration Assay

HRECs were seeded at 8000 cells/well on the upper layer of semipermeable membrane of a Transwell insert placed in a 24-well plate. The semipermeable membrane was coated with extracellular matrix to allow cell adhesion and migration. Culture medium was added to the lower chamber beneath the semipermeable membrane. According to the above groupings, incubation was continued for 24 h, then HRECs that migrated through the semipermeable membrane were stained and counted. Data were recorded and analyzed for the inhibitory effect on the migration ability of HRECs.

### 1.2 Protein Expression Levels of Inflammatory Factors and Neovascularization-Related Factors

Proteins extracted from HRECs after 24-hour treatment in each experimental group were quantified using the BCA method. Equal amounts of protein were thoroughly mixed with a loading buffer, denatured, and then subjected to electrophoresis using 7.5%-12.5% SDS-PAGE gel, transfer, blocking, antibody incubation, and development. The expression levels of target proteins were detected to evaluate their impact on the protein expression levels of inflammatory factors and neovascularization-related factors.

### 2. Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results

### 3.1 sIVs_{RGD}@PEDF Inhibited VEGF-Induced Endothelial Cell Proliferation - CCK Assay

The CCK assay is a commonly used method for detecting cell proliferation. The results demonstrated that sIVs alone could not significantly inhibit endothelial cell proliferation. In contrast, sIVs_{RGD}@PEDF could significantly inhibit endothelial cell proliferation. Although no statistically significant difference was observed compared to sEVs_{RGD}-PEDF, sIVs_{RGD}@PEDF showed a significant inhibitory effect on HRECs proliferation compared to sIVs_{GFP}@PEDF (FIG. 23).

### 3.2 sIVs_{RGD}@PEDF Inhibited VEGF-Induced Endothelial Cell Migration - Transwell Assay

The Transwell assay is a commonly used method for detecting cell migration. The results demonstrated that sIVs alone could not significantly inhibit endothelial cell migration. In contrast, sIVs_{RGD}@PEDF could significantly inhibit endothelial cell migration. Although no statistically significant difference was observed compared to sEVs_{RGD}-PEDF, sIVs_{RGD}@PEDF showed a significant inhibitory effect on HRECs migration compared to sIVs_{GFP}@PEDF (FIG. 24).

### 3.3 sIVs_{RGD}@PEDF Inhibited the Release of Inflammatory Factors in VEGF-Induced Endothelial Cells

Intercellular cell adhesion molecule-1 (ICAM-1), a member of the immunoglobulin superfamily of adhesion molecules, is an important adhesion molecule mediating adhesive reactions. ICAM-1 is expressed at low levels in quiescent endothelial cells and is highly expressed in VEGF-induced endothelial cells.

By collecting proteins from endothelial cells to detect the expression level of ICAM-1, the results showed that sIVs_{RGD}@PEDF significantly reduced the expression level of ICAM-1, showing a more pronounced therapeutic effect compared to PEDF alone and sIVs_{RGD}. Furthermore, sIVs_{RGD}@PEDF and sEVs_{RGD}@PEDF exhibited equivalent capabilities for inhibiting ICAM-1 (FIG. 25), indicating that in *in vitro* experiments, sIVs_{RGD}@PEDF and sEVs_{RGD}@PEDF exhibited equivalent anti-inflammatory effects.

### 4. Summary

sIVs_{RGD}@PEDF inhibited VEGF-induced endothelial cell proliferation and migration, showing a significant inhibitory effect on HRECs proliferation compared to sIVs-GFP, PEDF, and sIVs_{GFP}@PEDF, with no statistically significant differences in efficacy compared to sEVs_{RGD}@PEDF.

### Example 13: In Vivo Experiments of sIVs_{RGD}@PEDF Composite Formulation

### 1. Experimental Methods

### 1.1 Intravitreal Injection of OIR Mice

The OIR mouse model was established as described previously. Intravitreal injection was performed at P12. Each eye was injected with 1 µL of liquid. Groupings included PBS, sIVs-GFP, PEDF, sIVs_{GFP}@PEDF, sIVs_{RGD}@PEDF, sEVs_{RGD}@PEDF, with each vesicle source referring to Example 9. Subsequent observation and evaluation were performed.

### 1.2 Retinal Flat-Mount Staining and Analysis of Neovascularization Area and Non-Perfusion Area

Retinal flat mounts were performed at P17. Mouse eyeballs were enucleated, fixed in paraformaldehyde at room temperature for 15 min, and placed in cold PBS for 5-10 min. The eyeballs were transferred to gauze. Under an operating microscope, an incision was made at the limbus. The sclera was gently peeled off using two toothed forceps. The lens was removed. The retina was cut into four petals with small scissors, and obvious vascular clusters were cut off. Cold anhydrous methanol was gently dropped onto the retina for fixation. After the retina turned white, it was transferred to a 2 mL flat-bottom EP tube. The anhydrous methanol in the EP tube was aspirated. The retina was washed twice with PBS on a 4°C shaker, added with 1 mL of blocking buffer, incubated on a 4°C shaker for 2 h, washed twice with PBS, added with 200 µL of IB4, incubated overnight on a 4°C shaker in the dark, washed five times with PBS with 1 h for each time, and mounted after washing. The stained retina was photographed under a confocal microscope. The areas of retinal neovascularization and non-perfusion were annotated and quantified using Photoshop software. Significant statistical differences between groups were analyzed.

### 1.3 Electroretinography (ERG)

Retinal nerve function in mice at P25 (±1 d) and P42 (±1 d) was assessed using an electroretinography instrument to evaluate retinal function in OIR mice. Mice were dark-adapted overnight and anesthetized before ERG. The cornea was anesthetized with 0.5% proparacaine, and gatifloxacin eye gel was administrated to the ocular surface to prevent tissue drying. A reference electrode was inserted under the scalp near the midline between the ears. Another electrode was inserted into the tail of mouse. Light intensity stimulation parameters were set in the range of -1.1, 0.1, 1.0, and 3.0 log (cd•s/m²). Each mouse was tested three times to obtain an average value. The a-wave amplitude was measured from the baseline to the trough of the a-wave. The b-wave amplitude was measured from the peak of the a-wave to the peak of the b-wave, with automatic quantification using ERG software.

### 1.4 Protein Expression Levels of Inflammatory Factors and Neovascularization-Related Factors

Proteins extracted from the retinas of mice at P17 in the above experimental groups were quantified using the BCA method. Equal amounts of protein were thoroughly mixed with a loading buffer, denatured, and then subjected to electrophoresis using 7.5%-12.5% SDS-PAGE gel, transfer, blocking, antibody incubation, and development. The expression levels of target proteins were detected to evaluate their impact on the protein expression levels of inflammatory factors and neovascularization-related factors.

### 2. Statistical Analysis

Experimental data were expressed as mean ± standard deviation ( *x̅* ± *s* ). All experimental data were subjected to normality tests. All quantitative data were analyzed using SPSS 22.0. Analysis of variance was performed using One-way ANOVA, and post-hoc test was conducted using the least significant difference (LSD) analysis. For nonnormally distributed data and data with unequal variance, non-parametric tests were used, with P-value <0.05 considered as statistically significant difference.

### 3. Experimental Results3.1 sIVs_{RGD}@PEDF Significantly Reduced Retinal Non-Perfusion Area and Inhibited Neovascularization in OIR Mice

OIR mice were intravitreally injected with different concentrations of sIVs_{RGD}@PEDF and sEVs_{RGD}@PEDF, retinal flat mounts were prepared at P17, and the retinal non-perfusion area and neovascularization area were statistically analyzed. The results showed that sIVs_{RGD}@PEDF inhibited the formation of retinal non-perfusion area and neovascularization at low (0.5 mg/mL), medium (1 mg/mL), and high (2 mg/mL) concentrations. The therapeutic effect of the medium concentration was more pronounced than that of the low concentration, while no statistically significant difference was observed compared to the high concentration. Therefore, the medium concentration of sIVs_{RGD}@PEDF and sEVs_{RGD}@PEDF was selected for subsequent experiments (FIG. 26).

By performing retinal flat mounts on mice from each treatment group and quantifying the retinal non-perfusion area and neovascularization area, the results showed that sIVs_{RGD}@PEDF significantly inhibited the formation of retinal non-perfusion area and neovascularization, exhibiting a therapeutic effect equivalent to that of sEVs_{RGD}@PEDF, and demonstrating a superior therapeutic effect compared to the non-targeting sIVs_{GFP}@PEDF (FIG. 27).

### 3.2 sIVs_{RGD}@PEDF Exhibited Stronger Neuroprotective Effect Than sEVs_{RGD}@PEDF

We used ERG to evaluate the retinal electrophysiological function of OIR mice at P42. The results showed that the ERG amplitudes of a-wave and b-wave in the untreated OIR mice were reduced (Panel A and C of FIG. 28), indicating decreased function of bipolar cells and photoreceptor cells. Statistical analysis revealed that across all tested flash intensities, the light response of OIR mice was significantly reduced. Treatments with sIVs, sIVs_{GFP}@PEDF, sIVs_{RGD}@PEDF, and sEVs_{RGD}@PEDF significantly rescued the retinal light response of retinas (FIG. 28). Furthermore, the improvement in a-wave amplitude and b-wave amplitude under the highest stimulus by sIVs_{RGD}@PEDF was significantly superior to that by sEVs_{RGD}@PEDF and sIVs_{GFP}@PEDF (Panel B and D of FIG. 28). In summary, our findings indicated that sIVs_{RGD}@PEDF exhibited a stronger neuroprotective effect than sEVs_{RGD}@PEDF and sIVs_{GFP}@PEDF.

### 3.3 sIVs_{RGD}@PEDF Exhibited Stronger Ability to Inhibit Inflammation than sEVs_{RGD}@PEDF

Glial fibrillary acidic protein (GFAP) is a marker for astrocyte activation and serves as an important retinal inflammation-related factor. By collecting retinal proteins from OIR mice at P17 and detecting the expression level of GFAP, the results showed that sIVs_{RGD}@PEDF significantly reduced the expression level of GFAP, showing a more pronounced therapeutic effect compared to the non-targeting sIVs_{GFP}@PEDF. Moreover, the ability of sIVs_{RGD}@PEDF to inhibit GFAP expression was stronger than that of sEVs_{RGD}@PEDF, indicating that sIVs_{RGD}@PEDF offered superior therapeutic efficacy over the non-targeting one, and that sIVs_{RGD}@PEDF exhibited stronger ability to inhibit inflammation than sEVs_{RGD}@PEDF (Panel A and B of FIG. 29).

Vascular endothelial growth factor (VEGF) is a highly specific growth factor that promotes the growth of vascular endothelial cells, exhibiting functions of promoting vascular permeability, migration, proliferation, and angiogenesis of endothelial cells, making it an important neovascularization-related factor. By collecting retinal proteins from OIR mice at P17 and detecting the expression level of VEGF, the results showed that sIVs_{RGD}@PEDF significantly reduced the expression level of VEGF, showing a more pronounced therapeutic effect compared to the non-targeting sIVs_{GFP}@PEDF. Furthermore, sIVs_{RGD}@PEDF and sEVs_{RGD}@PEDF exhibited equivalent ability to inhibit VEGF, indicating that the neovascularization-targeting sIVs_{RGD}@PEDF offered superior therapeutic efficacy over the non-targeting one, and that sIVs_{RGD}@PEDF and sEVs_{RGD}@PEDF possessed equivalent inhibitory effects on VEGF (Panel A and C of FIG. 29).

### 4. Summary

The therapeutic effect of sIVs_{RGD}@PEDF on OIR mice was dose-dependent. At a dose of 1 mg/mL, sIVs_{RGD}@PEDF significantly reduced the retinal non-perfusion area, inhibited neovascularization, inhibited retinal inflammation, and protected retinal structure and function in OIR mice. Moreover, its efficacy was stronger than that of sIVs_{GFP}@PEDF, which also possessed neovascularization-targeting ability. Notably, sIVs_{RGD}@PEDF exhibited stronger abilities in inhibiting inflammation and providing neuroprotection than sEVs_{RGD}@PEDF.

The foregoing descriptions are merely preferred embodiments of the present disclosure and are not intended to limit the present disclosure. Any modifications, equivalent substitutions, and the like made within the spirit and principles of the present disclosure shall be included within the scope of protection of the present disclosure.

The aforementioned embodiments and methods described in the present disclosure may vary based on the ability, experience, and preferences of those skilled in the art.

The mere listing of steps of a method in a certain order in the present disclosure does not constitute any limitation on the order of the method steps.

## Claims

1. A method for preparing an intracellular-derived targeted nanovesicle, comprising the following steps:
(1) introducing a nucleotide encoding a targeting molecule-Lamp2b fusion protein into a cell;
(2) dispersing a resulting cell obtained from step (1) in a suspending solvent and performing ultrasonic treatment;
(3) subjecting a resulting liquid obtained from step (2) to one or more rounds of centrifugation, discarding cell membrane and organelle fragments, and collecting a resulting supernatant; and
(4) subjecting the supernatant obtained from step (3) to ultracentrifugation, and collecting a resulting pellet as the intracellular nanovesicle.

2. The method according to claim 1, wherein the step (1) comprises: constructing a plasmid overexpressing the targeting molecule-Lamp2b fusion protein, packaging the plasmid into a lentivirus, and transfecting the cell with the lentivirus.

3. The method according to claim 1, wherein the targeting molecule is a polypeptide, an antibody, an activating or inhibitory receptor, a protein ligand, a biologically active enzyme, a nucleic acid drug, or a combination thereof.

4. The method according to claim 3, wherein the targeting molecule is the polypeptide.

5. The method according to claim 4, wherein the targeting molecule is an A25 polypeptide or an RGD polypeptide.

6. The method according to any one of claims 3-5, wherein the targeting molecule is located at N-terminus of the Lamp2b.

7. The method according to claim 1, wherein in the step (2), an amplitude of the ultrasonic treatment is 20%-25%; and/or the ultrasonic treatment is performed for 10 s -20 s.

8. The method according to claim 7, wherein in the step (2), the amplitude of the ultrasonic treatment is 20%; and/or the ultrasonic treatment is performed for 15 s, with a cycle of 2 s on and 2 s off.

9. The method according to claim 1, wherein in the step (3), the centrifugation is performed twice, with respective parameters as follows:
1000 g -3000 g for 5 minutes -20 minutes;
10000 g -30000 g for 20 minutes -40 minutes; and
in the step (4), parameters of the ultracentrifugation comprise 100000 g -180000 g for 50 minutes -100 minutes.

10. The method according to claim 1, wherein the cell is at least one of a stem cell, a cardiomyocyte, a human embryonic kidney cell, a macrophage, a T cell, and a tumor cell.

11. The method according to claim 10, wherein the stem cell is a mesenchymal stem cell, the mesenchymal stem cell comprises an umbilical cord mesenchymal stem cell, a bone marrow mesenchymal stem cell, an adipose-derived mesenchymal stem cell, a dental pulp mesenchymal stem cell, or mesenchymal stem cells derived from placenta, amniotic fluid, and amnion.

12. A vesicle prepared by the method according to any one of claims 1-11.

13. The vesicle according to claim 12, wherein the vesicle is an intracellular nanovesicle modified by A25-Lamp2b overexpression or an intracellular nanovesicle modified by RGD-Lamp2b overexpression.

14. Use of the vesicle prepared by the method according to any one of claims 1-11 as a drug carrier in the preparation of a drug for preventing and/or treating a disease.

15. The use according to claim 14, wherein the disease is an ocular disease.

16. The use according to claim 15, wherein the disease is autoimmune uveitis or a retinal neovascular disease.

17. A method for preparing a drug-loaded vesicle, comprising using the vesicle prepared by the method according to any one of claims 1-11 as a drug carrier for drug loading.

18. A drug-loaded vesicle comprising a drug carrier, wherein the drug carrier is the vesicle prepared by the method according to any one of claims 1-11.
